# EUROPEAN PATENT APPLICATION

(11) **EP 4 015 626 A1**
(43) Date of publication of application: **22.06.2022**
(21) Application number: 20215768.1
(22) Date of filing: 18.12.2020
(51) Int. Cl.: C12N 9/10, C12P 5/00, C12P 7/02, C12P 7/62

(54) **ENZYMES AND METHODS FOR FERMENTATIVE PRODUCTION OF MONOTERPENE ESTERS**

(71) Applicant: Isobionics B.V., 6167 RD Geleen (NL)
(72) Inventor: BEEKWILDER, Martinus Julius, 6167 RD Geleen (NL); STYLES, Matthew Quinn, 6167 RD Geleen (NL); VOS, Aurin Minnert, 6708 PB Wageningen (NL); HESSELINK, Thamara, 6708 PB Wageningen (NL); BOSCH, Hendrik Jan, 6708 PB Wageningen (NL)
(74) Representative: Altmann Stößel Dick Patentanwälte PartG mbB

(57) **Abstract**

The present invention relates to an alcohol acyl transferase which is capable of esterifying a tertiary monoterpene alcohol such that at least 30% by mass of said tertiary monoterpene alcohol is esterified, preferably within 36 h, 24 h, 18 h, 12 h, 6 h, 3 h, 2 h, 1h, 45 min or 30 min, more preferably in a microbial cell. The invention further relates to a nucleic acid comprising a nucleic acid sequence encoding the alcohol acyl transferase of the invention, or a complementary sequence thereof, and a vector or gene construct comprising the nucleic acid of the invention. Further provided by the present invention is a host cell comprising the vector or gene construct of the invention, and a transgenic non-human organism comprising the nucleic acid of the invention, the vector or gene construct of the invention, or the host cell of the invention. The invention also concerns a method for preparing a monoterpene ester, comprising esterifying a monoterpene alcohol to a monoterpene ester, in the presence of an alcohol acyl transferase the invention. Specifically, it provides a method for preparing linalyl acetate, comprising esterifying linalool to linalyl acetate, in the presence of an alcohol acyl transferase of the invention. The invention further pertains to the use of the alcohol acyl transferase of the invention, the nucleic acid of the invention, the vector or gene construct of the invention, the host cell of the invention, or the transgenic non-human organism of the invention (i) for heterologous reconstitution of a terpene biosynthetic pathway; (ii) for producing an industrial product, preferably a flavour or fragrance, a biofuel, a fuel composition, a fuel compound, e.g., a blowing agent for diesel fuel compositions, a pesticide, an insect repellent or an antimicrobial; (iii) for producing an aliphatic and/or aromatic monoterpene ester from a monoterpene alcohol, preferably from a tertiary monoterpene alcohol; (iv) for detoxifying a monoterpene alcohol in a microorganism, thereby increasing monoterpene production in said microorganism; (v) in combination with a GPP synthase and/or S- or R- linalool synthase; (vi) for increasing the beneficial effects of acetylation in that the hydrophobic acetate partitions more readily go into an organic phase, as compared to the monoterpene alcohol; (vii) for expressing the alcohol acyl transferase of the invention such that the ratio of monoterpene acetate to monoterpene alcohol is greater than 5:1 or 10:1 or (viii) in a microbial production system for monoterpene esters. The invention also provides a kit comprising the alcohol acyl transferase of the invention, the nucleic acid of the invention, the vector or gene construct of the invention, the host cell of the invention, or the transgenic non-human organism of the invention, and optionally at least one monoterpene alcohol, preferably a tertiary monoterpene alcohol. Finally, the invention relates to a method for the production of a fuel and/or biolubricant compound, wherein the method comprises the steps of: a) Producing one or more monoterpene esters by any one of the methods of the invention; b) optionally, purifying the one or more monoterpene esters produced in step a); and c) converting the one or more monoterpene esters of step a), or the optionally purified one or more monoterpene esters of step b), to a fuel and / or biolubricant compound.

## Description

The present invention relates to an alcohol acyl transferase which is capable of esterifying a tertiary monoterpene alcohol such that at least 30% by mass of said tertiary monoterpene alcohol is esterified, preferably within 36 h, 24 h, 18 h, 12 h, 6 h, 3 h, 2 h, 1h, 45 min or 30 min, more preferably in a microbial cell. The invention further relates to a nucleic acid comprising a nucleic acid sequence encoding the alcohol acyl transferase of the invention, or a complementary sequence thereof, and a vector or gene construct comprising the nucleic acid of the invention. Further provided by the present invention is a host cell comprising the vector or gene construct of the invention, and a transgenic non-human organism comprising the nucleic acid of the invention, the vector or gene construct of the invention, or the host cell of the invention. The invention also concerns a method for preparing a monoterpene ester, comprising esterifying a monoterpene alcohol to a monoterpene ester, in the presence of an alcohol acyl transferase the invention. Specifically, it provides a method for preparing linalyl acetate, comprising esterifying linalool to linalyl acetate, in the presence of an alcohol acyl transferase of the invention. The invention further pertains to the use of the alcohol acyl transferase of the invention, the nucleic acid of the invention, the vector or gene construct of the invention, the host cell of the invention, or the transgenic non-human organism of the invention (i) for heterologous reconstitution of a terpene biosynthetic pathway; (ii) for producing an industrial product, preferably a flavour or fragrance, a biofuel, a fuel composition, a fuel compound, e.g., a blowing agent for diesel fuel compositions, a pesticide, an insect repellent or an antimicrobial; (iii) for producing an aliphatic and/or aromatic monoterpene ester from a monoterpene alcohol, preferably from a tertiary monoterpene alcohol; (iv) for detoxifying a monoterpene alcohol in a microorganism, thereby increasing monoterpene production in said microorganism; (v) in combination with a GPP synthase and/or S- or R- linalool synthase; (vi) for increasing the beneficial effects of acetylation in that the hydrophobic acetate partitions more readily go into an organic phase, as compared to the monoterpene alcohol; (vii) for expressing the alcohol acyl transferase of the invention such that the ratio of monoterpene acetate to monoterpene alcohol is greater than 5:1 or 10:1 or (viii) in a microbial production system for monoterpene esters. The invention also provides a kit comprising the alcohol acyl transferase of the invention, the nucleic acid of the invention, the vector or gene construct of the invention, the host cell of the invention, or the transgenic non-human organism of the invention, and optionally at least one monoterpene alcohol, preferably a tertiary monoterpene alcohol. Finally, the invention relates to a method for the production of a fuel and/or biolubricant compound, wherein the method comprises the steps of: a) Producing one or more monoterpene esters by any one of the methods of the invention; b) optionally, purifying the one or more monoterpene esters produced in step a); and c) converting the one or more monoterpene esters of step a), or the optionally purified one or more monoterpene esters of step b), to a fuel and / or biolubricant compound.

During the past few decades, intense scientific research focused on the most abundant secondary metabolites in all living organisms, the terpenes. More than 55,000 terpenoid substances are widely distributed among different families of natural products found in all biological kingdoms.

Many terpenoids are secondary metabolites as they are commonly, not primarily, essential for growth, development, or reproduction of any organism. However, this classification does not expand on the broad additional effects of these secondary metabolites that keep an ecosystem functioning. These substances play important roles and may provide plants with evolutionary advantages in relation to their distinct chemosensory properties such as smell. Amongst others, they may exert insecticidal effects thus protecting plants and crops against pests and pathogens, or may act as pollinator attractants in reproductive processes.

Many terpenoids are renowned for their economic importance being widely used as base structural moiety in the production of drugs, flavours, fragrances, pigments, and disinfectants. For example, the monoterpene alcohol linalool which is the main essential oil constituent of rosewood, Aniba rosaeodora, is among the most frequently used ingredients in perfume production. In addition, the sesquiterpene lactone, artemisinin, extracted from the shrub Artemisia annua, is used in the first-line treatment of malaria. The tricyclic diterpene taxol, isolated from the bark of the Pacific yew tree, Taxus brevifolia, and its structural analogs, are used as anticancer agents.

Terpenes are primarily synthesized in plants via common biosynthetic routes. In spite of their diverse structures and functions, all terpenes are built up of isoprene units (five- carbon atoms) following the isoprene rule. According to the number of isoprene units in their structure which are connected through head-to-tail addition, terpenes are classified according to their number of carbon atoms or sesquiterpenoid moieties, respectively: monoterpenes (C10), sesquiterpenes (C15), diterpenes (C20), triterpenes (C30), or polyterpenes having up to 30,000 connected isoprene units. Just like terpenes, terpenoids are likewise classified according to the number of isoprene units they are constituted of and are further named with the suffix -oids, as in monoterpenoids (C10), or in sesquiterpenoids (C15).

Isopentyl diphosphate (IPP) and its electrophilic isomer, dimethylallyl diphosphate (DMAPP), are the universal precursors in the biosynthesis of terpenes. Starting from these two building blocks, linear prenyl diphosphates are synthesized by a group of enzymes belonging to the prenyltransferases. IPP and DMAPP are condensed by the catalytic effect of the prenyltransferase geranyl diphosphate synthase to give the C10 geranyl diphosphate (GPP), the intermediate that can be converted to cyclic or linear end-products, representing the group of monoterpenes.

Similarly, sesquiterpenes are generated via the addition of a third isoprene unit to GPP forming the C15 farnesyl diphosphate (FPP), the biosynthetic precursor of common sesquiterpenes. Further polymerization of IPP and DMAPP produces longer prenyl diphosphates forming different classes of terpenes named according to the number of contained isoprene units.

IPP and DMAPP biosynthesis is accomplished via two independent pathways: the mevalonic acid (MVA) pathway and the 2-C-methyl-D-erythritol 4-phosphate (MEP) pathway. Although the MVA pathway was considered the universal route in the synthesis of terpenes, it was found to be less prominent in plant secondary metabolites than the MEP pathway during the last decades. MVA is dominant in most eukaryotes, archaea, few eubacteria as well as the cytosol and mitochondria of plants, and generates the precursors for sesquiterpenes (C15) and multiplied analogues such as triterpenes (C30), within the cytoplasm. On the other hand, the MEP pathway is the primary route in chloroplasts of higher plants, cyanobacteria, eubacteria, and algae. With its biosynthetic location in the plastids, MEP leads to monoterpenes (C10), diterpenes (C20) and carotenoids (C40).

The Mevalonic Acid Pathway (MVA) pathway, also known as mevalonate pathway, isoprenoid pathway, or 3- hydroxy-3-methylglutaryl-CoA (HMG-CoA) reductase pathway, was discovered in yeasts and animals in the 1950s. The MVA pathway starts with the Claisen condensation of two acetyl CoA molecules to form the acetoacetyl CoA through the catalytic action of the acetoacetyl CoA transferase enzyme. Acetoacetyl CoA is converted, via an aldol reaction with another acetyl CoA, to HMG-CoA by HMG synthase. In the next two reduction steps, two nicotinamide adenine dinucleotide phosphate molecules are required to convert HMG-CoA to mevalonic acid (MVA) with the help of HMG-CoA reductase. Subsequent phosphorylation of MVA gives mevalonate-5-diphosphate (MVAPP) via two reactions catalyzed by mevalonic acid kinase (MK) and phosphomevalonate kinase (PMK), respectively. Finally, IPP is produced from decarboxylation of MVAPP by an ATP-coupled decarboxylation reaction catalyzed by mevalonate-5-diphosphate decarboxylase (MVD). The IPP:DMAPP isomerase (IDI) then catalyzes the interconversion between IPP and DMAPP.

The Methylerythritol Phosphate Pathway (MEP), or the MVA-independent pathway, was discovered in bacteria and the chloroplasts of green algae and higher plants by Rohmer, Arigoni, and others, in the late 1990s and early 2000s. This pathway starts with two different precursors, namely pyruvate and D-glyceraldehyde 3-phosphate (G3P). Both molecules undergo condensation catalyzed by 1-deoxy-D-xylulose 5-phosphate synthase (DXS) yielding the 1-deoxy-D-xylulose 5-phosphate (DXP), using thiamine pyrophosphate as a cofactor. In the next step, DXP is isomerized by DXP reducto-isomerase (DXR) to MEP. 4-Diphosphocytidyl-2-C-methyl-D-erythritol (CDP-ME) synthase catalyzes, consequently, the coupling between MEP and cytidine triphosphate (CTP), producing methylerythritolcytidyl diphosphate (CDP-ME). In an ATP-dependent reaction, CDP-ME kinase phosphorylates CDP-ME to 4-diphosphocytidyl-2-C-methyl-D-erythritol-2-phosphate (CDP-MEP). Subsequently, the latter undergoes cyclization to 2-C-methyl-D-erythritol-2,4-cyclodiphosphate (MEcPP), in a reaction catalyzed by MEcPP synthase, releasing cytidine monophosphate (CMP). The pathway ends up by ring opening of the cyclic pyrophosphate and the reductive dehydration of MEcPP to 4-hydroxy-3- methylbut-2-enyl-diphosphate (HMBPP) being catalyzed by HMBPP synthase. HMBPP is finally converted by HMBPP reductase to a mixture of IPP and DMAPP.

In recent years, a number of terpenes including monoterpenes, sesquiterpenes, and their alcohols have been produced in microbial systems, in order to provide alternatives for terpenes from plant sources. Most commercially available terpenes are made by chemical synthesis, or by extraction from plant material. Plant sources are often compromised by low concentrations, harvest dependency, presence of pesticides, and/or risk of extinction of the plant species. Biotechnological production of terpenes can provide sustainable and economically viable alternatives for plant sources. Examples of such biotechnological production methods include valencene, artemisinic acid, and sclareol.

Many terpenes can be produced by microbial biotechnology. Esters such as ethyl acetate can be produced by yeasts such as Kluyveromyces marxianus. These yeasts use an EAT protein from the α/β hydrolase family to convert ethanol and acetyl CoA to ethyl acetate (e.g., Kruis et al., (2019) Microbial production of short and medium chain esters: Enzymes, pathways, and applications. Biotechnology Advances, 37 (7), 107407). Activity of these EAT proteins on more complex molecules has not been investigated into much detail.

Producing monoterpene esters in microorganisms has also been demonstrated. When geraniol was produced in E. coli, it was observed that the chloramphenicol acetyltransferase gene mediated formation of geranyl acetate (Liu et al. Biotechnol Biofuels (2016) 9:58). The use of more specific enzymes has been shown to bring advantages: While monoterpene alcohols such as geraniol, but also linalool which is an acyclic monoterpene found in the floral scents of many plants, are very toxic to microbes, their esters are often much less toxic. Toxicity of monoterpene alcohols often leads to an arrest in growth and/or production, and thus only very low product titers have been achieved. Chacon et al. have shown that expression of RhAAT, in an E. coli which was engineered to produce geraniol, lead to formation of geranyl acetate at levels which were substantially increased relative to the levels of geraniol produced in the absence of RhAAT (Chacón, M.G., et al. Esterification of geraniol as a strategy for increasing product titre and specificity in engineered Escherichia coli. Microb Cell Fact 18, 105 (2019); https://doi.org/10.1186/s12934-019-1130-0; WO 2019/092388). For that reason, in situ esterification of monoterpene alcohols such as geraniol has been forwarded as a means to detoxify the product and, thus, increase terpene production.

In plants, esters are made by using alcohol acyl transferases (AATs) from the BAHD family of proteins, which couple an alcohol to an acyl-CoA. The BAHD acyl transferase superfamily was named according to the first four biochemically characterized enzymes of this family in plants: benzylalcohol O-acetyltransferase (BEAT), anthocyanin O-hydroxy-cinnamoyltransferase (AHCT), anthranilate N-hydroxy-cinnamoyl/benzoyl transferase (HCBT) and deacetylvindoline 4-O-acetyltransferase (DAT). Members of the BAHD family share two conserved motifs: the HxxxD motif which is involved in catalysis and the DFGWG motif whose role is still unclear (Bayer A et al. (2004) Bioorg Med Chem 12, 2787-2795).

Well known examples of alcohol acyl transferases (AATs) include AATs from fruit such as strawberry and banana, which typically produce fruit esters from aliphatic alcohols, such as octanol, hexanol or isoamylalcohol (Aharoni et al., 2000 Identification of the SAAT gene. Plant Cell. 2000 May;12(5):647-62. doi: 10.1105/tpc.12.5.647.; Beekwilder et al., (2004) Functional characterization of enzymes forming volatile esters from strawberry and banana. Plant Physiol. 2004 135(4):1865-78. doi: 10.1104/pp.104.042580.).

Alcohol acyl transferases (AATs) also mediate esterification of more complex molecules: For example the Salutaridinol 7-O-Acetyltransferase mediates a step in morphine biosynthesis, using a complex isoquinoline alkaloid as substrate (Grothe et al., 2001, DOI 10.1074/jbc.M102688200), as part of a series of at least 17 enzymatic steps. The Acetyl-CoA:deacetylvindoline 4-O-acetyltransferase catalyzes the last step in biosynthesis of vindoline, an indole alkaloid.

Monoterpene alcohols can also be used as substrates for alcohol acyl transferases (AATs). For example, the RhAAT from rose (Rosa hybrida) mediates geranyl acetate formation (Shalit et al., Volatile ester formation in roses. Identification of an acetyl-coenzyme A. Geraniol/Citronellol acetyltransferase in developing rose petals. Plant Physiol. 2003 Apr;131(4):1868-76. doi: 10.1104/pp.102.018572.). AATs from Lavandula have been described to mediate lavandulyl acetate formation (Sarker & Mahmoud (2015) Cloning and functional characterization of two monoterpene acetyltransferases from glandular trichomes of L. x intermedia. Planta 242, 709-719).

The rose AAT (RhAAT), but also many other alcohol acyl transferases (AATs) (e.g., strawberry AAT SAAT), are not very selective and accept a range of alcohol substrates, including phenylethanol, cinnamoyl alcohol, hexanol, isoamylalcohol and longer chain alcohols. However, linalool is not among the accepted substrates of these enzymes. The exceptional property of linalool, defining it as a non-substrate for these AATs, could be caused by the position of the alcohol group in linalool, which can be regarded as a tertiary alcohol: The carbon to which the acceptor alcohol group is attached is bonded to three carbon groups. Tertiary alcohols are often difficult to access by enzyme active site pockets, possibly because of the steric accessibility of the alcohol group.

Linalyl acetate is an acetyl ester formed from linalool. So far, linalyl acetate is produced chemically, and no fermentative large-scale processes for producing linalyl acetate have been reported yet; see, e.g., https://de.wikipedia.org/wiki/Linalylacetat. Linalyl acetate is an important flavour and fragrance molecule. However, an alcohol acyl transferase which is capable of esterifying linalool or other tertiary monoterpene alcohols in economically significant amounts has not yet been identified. In a recent study, trace activity on linalool has been reported for a BAHD acyl transferase from common thyme (Thymus vulgaris), when transiently expressed in Nicotiana benthamiana (doctoral thesis "Die Biosynthese von acetylierten Monoterpenen in Thymian (Thymus vulgaris)" by Natalie Wiese, June 2020; https://opendata.uni-halle.de/handle/1981185920/34248). However, such a trace activity with small side production of linalyl acetate in very specific experimental settings can disappear under conditions common in biocatalytic or biotechnological production on an industrial scale, or simply be unsuited for large-scale production or uneconomical for such use.

Consequently, producing linalyl acetate by biocatalysis or by biotechnological approaches is hindered by the lack of an alcohol acyl transferase which is capable of esterifying tertiary monoterpene alcohols, e.g. linalool, in economically significant quantities.

Thus, the technical problem underlying the present invention may be seen as the provision of means and methods complying with the aforementioned needs. The technical problem is solved by the embodiments characterized in the claims, herein below and the Examples.

The present invention relates to an alcohol acyl transferase which is capable of esterifying a tertiary monoterpene alcohol such that at least 30% by mass of said tertiary monoterpene alcohol is esterified, preferably within 36 h, 24 h, 18 h, 12 h, 6 h, 3 h, 2 h, 1 h, 45 min or 30 min, more preferably within a microbial cell.

Preferably, the alcohol acyl transferase (AAT) of the invention has alcohol acyl transferase activity as defined herein.

A "microbial cell" is defined elsewhere herein. Preferably, the microbial cell is an E. coli cell.

Preferred tertiary monoterpene alcohols include, but are not limited to, linalool, alpha terpineol, gamma terpineol, p-cymene-8-ol, p-menth-3-en-1-ol, p-menth-8-en-1-ol, 4-carvomenthol, and/or 4-Thujanol; see, e.g. Marnett et al. 2014, https://onlinelibrary.wiley.com/doi/full/10.1111/1750-3841.12407.

Preferably, the tertiary monoterpene alcohol comprises linalool and alpha terpineol. So the alcohol acyl transferase of the invention is advantageously capable of esterifying both linalool and alpha terpineol, in contrast to alcohol acyl transferases described in the art.

For instance, if linalool is esterifed by the alcohol acyl transferase of the invention, linalyl acetate is produced. If alpha terpineol is esterifed by the alcohol acyl transferase of the invention, alpha terpinyl acetate is generated.

Preferably, the alcohol acyl transferase of the invention is capable of esterifying the tertiary monoterpene alcohol linalool such that at least 30% by mass of said tertiary monoterpene alcohol linalool is esterified to linalyl acetate, preferably within 36 h, 24 h, 18 h, 12 h, 6 h, 3 h, 2 h, 1h, 45 min or 30 min. Preferably, the alcohol acyl transferase of the invention is capable of esterifying the tertiary monoterpene alcohol alpha terpineol such that at least 30% by mass of said tertiary monoterpene alcohol alpha terpineol is esterified to alpha terpinyl acetate, preferably within 36 h, 24 h, 18 h, 12 h, 6 h, 3 h, 2 h, 1h, 45 min or 30 min. More preferably, the alcohol acyl transferase of the invention is capable of esterifying the tertiary monoterpene alcohol linalool such that at least 30% by mass of said tertiary monoterpene alcohol linalool is esterified to linalyl acetate, and is further capable of esterifying the tertiary monoterpene alcohol alpha terpineol such that at least 30% by mass of said tertiary monoterpene alcohol alpha terpineol is esterified to alpha terpinyl acetate, preferably within 36 h, 24 h, 18 h, 12 h, 6 h, 3 h, 2 h, 1h, 45 min or 30 min.

Preferably, the alcohol acyl transferase of the invention is capable of esterifying a tertiary monoterpene alcohol such that at least 30% by mass of said tertiary monoterpene alcohol is esterified within the indicated preferred time period, in a microbial cell, such as a bacterial cell or a fungal cell. For instance, the alcohol acyl transferase of the invention is capable of esterifying a tertiary monoterpene alcohol such that at least 30% by mass of said tertiary monoterpene alcohol is esterified, when heterologously expressed, in bacteria, as shown in the Examples. More preferably, the bacteria are gram-negative bacteria, even more preferably the bacteria are of the genus Escherichia (e.g., E. coli) or Rhodobacter (e.g., Rhodobacter sphaeroides or Rhodobacter capsulatus).

Preferably, at least 40% by mass, 50% by mass, 60% by mass, 65% by mass, 70% by mass, 75% by mass, 78% by mass, 80% by mass, 90% by mass, or even 100% of said tertiary monoterpene alcohol, e.g. linalool and/or alpha terpineol is esterified, by the alcohol acyl transferase of the invention.

For determination of the content of the monoterpene ester produced by the alcohol acyl transferase of the invention, several instrumental techniques, such as gas chromatography-flame ionization detector (GC-FID), gas chromatography-mass selective detector (GC-MSD), high performance liquid chromatography-evaporative light scattering detector (HPLC-ELSD), high performance liquid chromatography-refractive index detector (HPLC-RID), high performance liquid chromatography-variable wavelength detector (HPLC-VWD), gel permeation chromatography (GPC), high performance thin layer chromatography (HPTLC), nuclear magnetic resonance (NMR), termogravimetric analysis (TGA), and infrared spectroscopy (IR) can be used which are known in the art; see, e.g., review by Jiang et al, Curr Protoc Plant Biol. 2016; 1: 345-358. doi:10.1002/cppb.20024. Preferably, GC-FID and GC-MSD are used, with NMR for additional characterization, for determination of the content of the monoterpene ester produced by the alcohol acyl transferase of the invention.

The alcohol acyl transferase of the invention can also be capable of esterifying a primary and/or a secondary monoterpene alcohol, in addition to a tertiary monoterpene alcohol. Monoterpene alcohols are defined herein below. For instance, (i) the alcohol acyl transferase of the invention is capable of esterifying a primary and a secondary and a tertiary monoterpene alcohol, (ii) it is capable of esterifying a primary and a tertiary monoterpene alcohol, or (iii) it is capable of esterifying a secondary and a tertiary monoterpene alcohol. Primary alcohols comprise, for example, geraniol, citronellol, lavandulol, perillyl alcohol, or thymol; secondary alcohols comprise, for instance, borneol, isoborneol, fenchol, verbenol, carveol, or menthol.

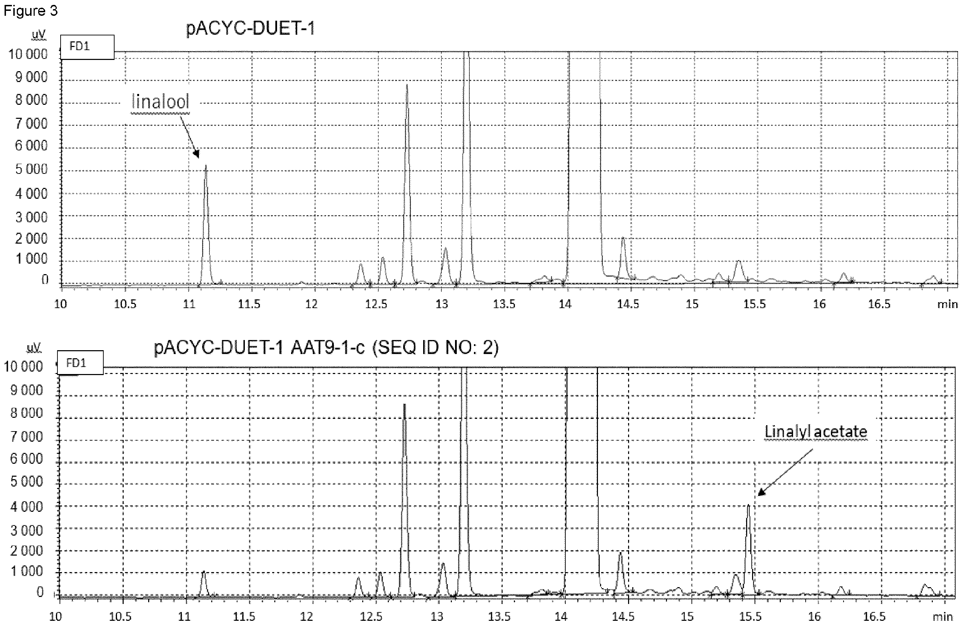

Advantageously, the present inventors could identify a novel alcohol acyl transferase (AAT) isolated from Citrus bergamia which accepts as substrate, a tertiary alcohol such as present in the monoterpene alcohol linalool or alpha terpineol. Specifically, linalool was converted to linalyl acetate when the alcohol acyl transferase of the invention, as exemplified by variant AAT9-1-c (SEQ ID NO: 2), was expressed. This is shown in Example 2 and Figure 3. This finding could not be expected, in view of the fact that none of the alcohol acyl transferase enzymes described in the prior art have been found to use linalool as a substrate, possibly due to the fact that tertiary alcohols are often difficult to access by enzyme active site pockets of alcohol acyl transferases (AATs). To the best knowledge of the present inventors, the only reported exception is an acyl transferase from common thyme (Thymus vulgaris) that showed very low catalytic activity towards linalool as a side activity, when transiently expressed in Nicotiana benthamiana (doctoral thesis "Die Biosynthese von acetylierten Monoterpenen in Thymian (Thymus vulgaris)" by Natalie Wiese, June 2020; https://opendata.uni-halle.de/handle/1981185920/34248; http://dx.doi.org/10.25673/34053). This enzyme is further discussed below. Having established that the alcohol acyl transferase of the present invention is active on linalool, a set of monoterpene alcohols and sesquiterpene alcohols was tested, in the presence of host cells expressing the alcohol acyl transferase of the invention, or empty vector pACYCDUET-1 as a control. Activity of the alcohol acyl transferase of the invention was surprisingly also observed on the tertiary monoterpene alcohol alpha terpineol. Further, the primary monoterpene alcohol geraniol, and the secondary monoterpene alcohol verbenol has been accepted as substrate, and corresponding esters could be observed. In contrast, the sesquiterpene alcohols nerolidol, patchoulol, (-)-alpha-bisabolol and cedrol were not observed to be converted to their corresponding esters.

In view of this finding, the alcohol acyl transferase of the invention as exemplified by AAT9-1-c (SEQ ID NO: 2) appears to be a monoterpene alcohol-specific acyltransferase: It accepts as substrate, primary, secondary and tertiary alcohols present in monoterpenes, but not the sesquiterpene alcohols nerolidol, patchoulol, (-)-alpha-bisabolol and cedrol.

In one embodiment, the monoterpene alcohol is is linalool, geraniol, alpha terpineol, gamma terpineol, lavandulol, fenchol, perillyl alcohol, menthol or verbenol, preferably linalool, alpha terpineol or perillyl alcohol, more preferably linalool or alpha terpineol.

Advantageously, the alcohol acyl transferase of the invention is capable of esterifying tertiary monoterpene alcohols, e.g., linalool and alpha terpineol, in economically significant quantities, and, thus, allows the production of monoterpene esters, e.g., linalyl acetate and alpha terpinyl acetate, in industrial scale.

The invention pertains to an alcohol acyl transferase capable of producing at least 50 µg, preferably at least 100 µg, more preferably at least 500 µg, even more preferably at least 1000 µg, yet even more preferably at least 1500 µg and most preferably at least 2000 µg of a monoterpene ester, preferably a monoterpene ester from a tertiary monoterpene alcohol, per minute and per gram of alcohol acyl transferase, at 30 °C, and at a pH in the range of 6.0 to 8.5, preferably at a pH value around pH 7.0, under conditions where the substrates are not limiting. The substrates are acyl-CoA and a monoterpene alcohol, preferably a tertiary monoterpene alcohol, as defined herein.

The alcohol acyl transferase of the invention has a Km value for linalool of at least 60 µM, preferably at least 100 µM.

More advantegously, the alcohol acyl transferase of the invention is capable of esterifying preferably linalool and/or alpha terpineol over geraniol, meaning that monoterpene alcohols other than geraniol, and preferably tertiary monoterpene alcohols, are the preferred substrates of the enzyme of the invention. The enzyme of the invention preferably has a higher affinity to linaool and/or alpha terpineol than to geraniol.

The invention hence further relates to an alcohol acyl transferase with a specific activity for at least one tertiary monoterpene alcohol, preferably linalool and/or alpha terpineol, that is at least 5 times, at least 10 times, at least 50 times, at least 100 times, at least 500 times or at least 1000 times higher than the specific activity of the same enzyme for geraniol. Preferably, the specific activity of the enzyme of the invention for linalool is at least 0.01 µmol, 0.05 µmol, 0.1 µmol, 0.5 µmol, 1.0 µmol, 2.0 µmol, 3.0 µmol, 4.0 µmol, 5.0 µmol, 10 µmol, 50 µmol or 100 µmol per nanogram enzyme and per hour.

This is in contrast to the alcohol acyl transferases described in the art which are either not capable of esterifying tertiary monoterpene alcohols at all, due to sterical hindrance, or show only trace activity on the tertiary monoterpene alcohol linalool, as the BAHD acyl transferase named TvAAT3 from common thyme (Thymus vulgaris), upon transient expressed in Nicotiana benthamiana (doctoral thesis "Die Biosynthese von acetylierten Monoterpenen in Thymian (Thymus vulgaris" by Natalie Wiese, June 2020; https://opendata.uni-halle.de/handle/1981185920/34248). The author herself refers to a "detectable but very low catalytic activity" of the BAHD acyl transferase TvAAT3 as regards linalool, in this particular transient expression system. Indeed, conversion of linalool was very low, because only less than 10% of linalool was converted. As the author could not find any conversion of linalool when she expressed the TvAAT3 in E. coli, she hypothesized that the action of a further enzyme is required for generating the presumed linalyl acetate in Nicotiana benthamiana; see page 73, second paragraph, of the mentioned doctoral thesis. Further, this very low catalytic activity on linalool in this specific transient expression system has only been confirmed by GC-MS (mass and retention index), and no formal structural assignment has been carried out to confirm the presence of linalyl acetate. As set forth above, BAHD acyl transferase TvAAT3 did not show any activity on linalool when expressed in E. coli (section 3.4.2, page 51, last sentence, of the doctoral thesis by Natalie Wiese), which is contrary to the alcohol acyl transferase of the invention; see Example 2. In addition, no alpha terpineol could be esterified, by the BAHD acyl transferase TvAAT3, neither in E. coli (section 3.4.2, page 51, last sentence, of the doctoral thesis by Natalie Wiese) nor in Nicotiana (section 3.4.3, page 53, last paragraph, of the doctoral thesis by Natalie Wiese). The amino acid sequence identity between TvAAT3 (doctoral thesis by Natalie Wiese, loc. cit) and AAT9-1-c (SEQ ID NO: 2) of the present application is 37.2 %.

In contrast to the reported BAHD acyl transferase TvAAT3, the novel alcohol acyl transferases of the invention show higher affinity, activity and improved turnover numbers as regards tertiary monoterpene alcohols in vitro and in vivo and produce monoterpene esters in host cells or non-human organisms that are amenable for large scale, industrial production, such as - but not limited to - E. coli and Rhodobacter sphaeroides.

Preferably, the alcohol acyl transferase (AAT) of the invention has an improved alcohol acyl transferase activity as regards a monoterpene alcohol, preferably a tertiary monoterpene alcohol, in comparison to alcohol acyl transferases (AAT) described in the art.

Preferably, said alcohol acyl transferase activity as regards a monoterpene alcohol, preferably a tertiary monoterpene alcohol, is at least 1.5 times, at least 2 times, at least 3 times, at least 4 times, at least 5 times, or even at least 10 times higher than the alcohol acyl transferase activity as regards said monoterpene alcohol, preferably a tertiary monoterpene alcohol, of an alcohol acyl transferase (AAT) known in the art, under identical test conditions.

More preferably, the alcohol acyl transferase of the invention has a turnover number with respect to a tertiary monoterpene alcohol that is at least 1.5 times, at least 2 times, at least 3 times, at least 4 times, at least 5 times, or even at least 10 times higher than the turnover number of said tertiary monoterpene by a known alcohol acyl transferase, under identical test conditions.

Preferably, the turnover number is an in vivo turnover number which means that the enzyme turnover number, kcat, is estimated in vivo; see, e.g., Heckmann et al., PNAS September 15, 2020 117 (37) 23182-23190; https://doi.org/10.1073/pnas.2001562117.

Preferably, the tertiary monoterpene alchohol is linalool or alpha terpeniol, or linalool and alpha terpineol.

Preferably, the alcohol acyl transferase (AAT) known in the art is the BAHD acyl transferase from common thyme (Thymus vulgaris) named TvAAT3 (doctoral thesis "Die Biosynthese von acetylierten Monoterpenen in Thymian (Thymus vulgaris)" by Natalie Wiese, June 2020; https://opendata.uni-halle.de/handle/1981185920/34248), and the compared alcohol acyl transferase activity is with respect to the tertiary monoterpene alcohol linalool, under identical test conditions.

The novel alcohol acyl transferases of the invention are especially useful for producing monoterpene esters, in particular when the monoterpene esters derive from tertiary monoterpene alcohols, as demonstrated in the Examples. For instance, they can be used for the production of linalyl acetate or other monoterpene esters indicated herein.

A BLASTP analysis of the variant AAT9-1-c sequence (SEQ ID NO: 2) against the SWISSPROT database of characterized proteins revealed that the closest characterized proteins are members of the vinorine synthase family; see Example 4. It could not be expected that this novel alcohol acyl transferase enzyme of the invention capable of esterifying tertiary monoterpene alcohols such as linalool, is most closely related to a family of alcohol acyl transferases (AAT) which acts on very complex phenolic substances, e.g. salutaridinol.
The present inventors reasoned that also in Lavandula, like in bergamot, members of the vinorine synthase family were candidates for linalool acetyl transferases. Indeed, they were able to identify linalool acetyltransferase homologues in Lavandula angustifolia, the amino acid sequences of which are depicted in SEQ ID NO: 3 (Lavandula AAT-10056) and SEQ ID NO: 4 (Lavandula AAT-1461); see Example 4.

Preferably, the alcohol acyl transferase of the invention is preferring a monoterpene alcohol over a sesquiterpene alcohol, as substrate. For instance, activity of the alcohol acyl transferase of the invention was found on the monoterpene alcohols geraniol, alpha terpineol and verbenol, and corresponding esters could be observed. In contrast, the sesquiterpene alcohols nerolidol, patchoulol, (-)-alpha-bisabolol and cedrol were not observed to be converted to their corresponding esters, by the enzyme of the invention. It is envisaged that the alcohol acyl transferase of the invention shows activity on other sesquiterpene alcohols but still prefers monoterpene alcohols over sesquiterpene alcohols, as substrates. Likely candidates include, for instance, nerolidol, patchoulol, bisabolol, germacrene D-ol, hedycariol, santalol, cubebol.

The alcohol acyl transferase of the invention can be manufactured by chemical synthesis or recombinant molecular biology techniques well known to the person skilled in the art, as shown in Example 1. This applies mutatis mutandis to the isolation of the alcohol acyl transferase of the invention from the host cell or supernatant; see, e.g., Sambrook et al., Molecular cloning: a laboratory manual / Sambrook, Joseph; Russell, David W. --. 3rd ed. -- New York: Cold Spring Harbor Laboratory, 2001; Ausubel, Current Protocols in Molecular Biology, Green Publishing Associates and Wiley Interscience, N.Y. (1994).

As used herein, the singular forms "a", "an" and "the" include both singular and plural reference unless the context clearly dictates otherwise. By way of example, "a primary monoterpene aclohol" refers to one or more than one primary monoterpene alcohol, "a secondary monoterpene aclohol" refers to one or more than one secondary monoterpene alcohol, "a tertiary monoterpene aclohol" refers to one or more than one tertiary monoterpene alcohol, or "a cell" refers to one or more than one cell.

As used herein, the term "about" when qualifying a value of a stated item, number, percentage, or term refers to a range of plus or minus 10 percent, 9 percent, 8 percent, 7 percent, 6 percent, 5 percent, 4 percent, 3 percent, 2 percent or 1 percent of the value of the stated item, number, percentage, or term. Preferred is a range of plus or minus 10 percent.

The terms "comprising", "comprises" and "comprised of" as used herein are synonyms with "including", "includes" or "containing", "contains", and are inclusive or open-ended and do not exclude additional, non-recited members, elements or method steps. Evidently, the term "comprising" encompasses the term "consisting of". More specifically, the term "comprise" as used herein means that the claim encompasses all the listed elements or method steps, but may also include additional, unnamed elements or method steps. For example, a method comprising steps a), b) and c) encompasses, in its narrowest sense, a method which consists of steps a), b) and c). The phrase "consisting of" means that the composition (or device, or method) has the recited elements (or steps) and no more. In contrast, the term "comprises" can encompass also a method including further steps, e.g., steps d) and e), in addition to steps a), b) and c).

In case numerical ranges are used herein such as "in a concentration between 1 and 5 micromolar", the range includes not only 1 and 5 micromolar, but also any numerical value in between 1 and 5 micromolar, for example, 2, 3 and 4 micromolar.

The term "in vitro" as used herein denotes outside, or external to, the animal or human body. The term "in vitro" as used herein should be understood to include "ex vivo". The term "ex vivo" typically refers to tissues or cells removed from an animal or human body and maintained or propagated outside the body, e.g., in a culture vessel. The term "in vivo" as used herein denotes inside, or internal to, the animal or human body.

The term "alcohol acyl transferase" (AAT) as used herein means an alcohol O-acetyltransferase. An alcohol O-acetyltransferase catalyses the reaction acetyl-CoA + an alcohol = CoA + an acetyl ester. So said enzyme catalyses an esterification step, transferring an acyl-CoA to an alcohol (see, e.g., EC 2.3.1.84). Accordingly, the term "alcohol acyl transferase activity" as used herein means catalysation of an esterification step, transferring an acyl-CoA to an alcohol group. Alcohol acyl transferases (AAT) are capable of combining different alcohols and acyl-CoAs that result in the synthesis of a wide range of esters, including monoterpene esters, evolved from fruit. Alcohol acyl transferases play a key role in ester biosynthesis. The diversity of volatile esters arises from the multiplicity of genes encoding alcohol acyl transferases with different specific substrate selectivity (Lucchetta et al., (2007) J Agric Food Chem 55, 5213-5220; D'Auria JC (2006), Curr Opin Plant Biol 9, 331-340).

As well known to those skilled in the art, enzymes bind to their substrates and transform them into products. A plot of the initial reaction velocity versus substrate concentration depicts a rectangular hyperbola. The reaction velocity (v) equals (Vmax [A])/(Km + [A]) as described by the Michaelis-Menten equation where Vmax is the maximal velocity, [A] is the substrate concentration, and Km is the Michaelis constant, or the substrate concentration at half maximal velocity. Steady-state enzyme kinetics are used to determine the Km value for substrates, the Vmax value for enzymes, and the Ki values for various inhibitors, including drugs.

The "turnover number" of an enzyme (kcat or catalytic rate constant) is the maximal number of molecules of substrate converted to product per active site per unit time of several different substrates to different products. The kcat/Km value, or specificity constant, of the various substrates can be compared. That substrate with the highest value is the best substrate for the enzyme, accounting for the name specificity constant. The rate of any reaction is limited by the rate at which reactant molecules collide. The diffusional limiting rate for a bimolecular reaction is 10⁸ to 10⁹ M⁻¹ s⁻¹. The ratio of kcat/Km is a first-order rate constant. The product of kcat/Km and the substrate concentration (at subsaturating levels) yields the rate of the enzyme-catalyzed reaction. This rate is proportional to the substrate concentration and is therefore designated first order. Enzymes that have ratios of kcat/Km near 10⁸ to 10⁹ M⁻¹ s⁻¹ (close to the maximum allowed by the rate of diffusion) have achieved catalytic perfection. For example, triose phosphate isomerase (EC 5.3.1.1), an enzyme of the glycolytic pathway, is an enzyme that has this attribute. Most enzymes, however, have specificity constants orders of magnitude below this value. Methods for determining the turnover number of an enzyme are well known in the art; see, e.g., https://doi.org/10.1016/B978-0-12-801238-3.05143-6 or Heckmann et al., PNAS September 15, 2020 117 (37) 23182-23190; https://doi.org/10.1073/pnas.2001562117. The term "linalool acetyl transferase" as used herein means an alcohol O-acetyltransferase which catalyses the reaction acetyl-CoA + linalool = CoA + linalyl acetate.

Tests for determining the activity of an alcohol acyl transferase as defined herein are well known in the literature (see, e.g., Chacón, M.G., et al. Esterification of geraniol as a strategy for increasing product titre and specificity in engineered Escherichia coli. Microb Cell Fact 18, 105 (2019); https://doi.org/10.1186/s12934-019-1130-0; WO 2019/092388; Aharoni et al., Identification of the SAAT gene. Plant Cell. 2000 May;12(5):647-62. doi: 10.1105/tpc.12.5.647.; Beekwilder et al., (2004) Functional characterization of enzymes forming volatile esters from strawberry and banana. Plant Physiol. 2004 135(4):1865-78. doi: 10.1104/pp. 104.042580; (Shalit et al., Volatile ester formation in roses. Identification of an acetyl-coenzyme A. Geraniol/Citronellol acetyltransferase in developing rose petals. Plant Physiol. 2003 Apr;131(4):1868-76. doi: 10.1104/pp.102.018572; Sarker & Mahmoud (2015) Cloning and functional characterization of two monoterpene acetyltransferases from glandular trichomes of L. x intermedia. Planta 242, 709-719). A suitable test for determining the activity of the alcohol acyl transferase of the invention is also shown in Example 2.

Per definition, the term "terpenes" comprises the hydrocarbons only, being composed of carbon and hydrogen. In contrast, the term "terpenoids" refers to terpenes containing additional functional groups, resulting in derivatives such as alcohols, aldehydes, ketones, and acids; see, e.g., Flavours and Fragrances: Chemistry, Bioprocessing and Sustainability RG Berger; Black et al., Terpenoids and their role in wine flavour: recent advances. Australian Journal of Grape and Wine Research 21, 582-600, 2015; Zhou & Pichersky, More is better: the diversity of terpene metabolism in plants. Current Opinion in Plant Biology 2020, 55:1-10 ; Degenhardt J, Köllner TG, Gershenzon J (2009) Monoterpene and sesquiterpene synthases and the origin of terpene skeletal diversity in plants. Phytochemistry 70(15):1621-1637). In the scientific literature, the term terpene is frequently used interchangeably with the term terpenoids, although they have different meanings. As used herein, the term "terpenes", comprises both hydrocarbons and their functionalized derivatives.

"Monoterpenes" as used herein are secondary metabolites in plants and the main constituents of essential oils; see, e.g., DH Grayson. Monoterpenoids. Nat Prod Rep 1996 Jun;13(3):195-225. doi: 10.1039/np9961300195. Thereby, they contribute to the specific smell characters of plants. Monoterpenes are characterised by their lipophilicity, low-molecular weight, and volatility. In plant tissue, they are primarily stored in the oil glands, glandular hairs, and trichomes of leaves. As mentioned elsewhere herein, C10 geranyl diphosphate (GPP) is the direct precursor in the formation of monoterpenes comprising a series of consecutive reactions including hydrolysis, cyclizations, and oxidoreductions.

There are two main types of monoterpenes: acyclic (or linear) and cyclic which can be mono- or bicyclic. Acyclic monoterpenes, such as cis-alpha-ocimene and beta-myrcene are 2,6-dimethyloctane derivatives. Typical monocyclic monoterpenes, as limonene and cymene, are, in principle, cyclohexane derivatives with an isopropyl substituent, commonly containing variable double bond moieties. Alpha-pinene and beta-pinene are, on the other hand, the common types of bicyclic monoterpenes.

Oxidation or rearrangement of the monoterpene structure is a biochemical modification that produces the so-called monoterpenoids. They may possess additional functional groups leading to substances with aldehydic, ketonic, ester, or alcohol base structures.

Monoterpenes, as most secondary metabolites, are crucial for the evolution of plants. Amongst others, they play an ecological role in plants in their defense against herbivores, bacteria, and fungi via monoterpene emission which attract, in return, the natural enemies of these herbivores. In addition, they mediate interactions between plants and their environment when acting as pollinator attractants or allelopathic agents.

In aromatic plants, isoprenes and especially monoterpenes, may provide a thermo-tolerance to their plants. Thus, plants secreting these terpenes can better tolerate short high- temperature periods of sunlight than non-terpene-emitting plants. Thereby, those emitting plants maintain high rates of photosynthesis compared to the non-emitting ones. Volatilized monoterpenes can further protect plants against oxidative damage due to their capability to react with oxidizing agents from the atmosphere. Monoterpenes are also found in oleoresins. Together with diterpene resin acids and sesquiterpenes, they act as chemical and physical defense compounds. This complex mixture, synthesized by plants, repels insects via intoxication and quenching. Moreover, they cure the plant wounds by forming a band-aid secure layer.

Monoterpenes are industrially used as flavour, fragrant, and cosmetic constituents. Fragrances and aromas are used as essential additives enhancing the final quality of foods and beverages, as well as in body care and other hygienic products. In recent time, there is a raising demand, however, for products of natural origin. Therefore, natural flavour compounds that can improve the sensory appeal of these products gained larger value and became more expensive than their artificial counterparts. Essential oils and their monoterpene constituents, originating from plants, are the main sources of flavours and fragrances, used in industry. One of the first known plants used in cosmetics is the peppermint plant, or Mentha piperita, which contains menthol as its main essential oil constituent. It is primarily applied because of its anti-stress, tension-relieving, and cooling effects. Menthol, one of the most renowned monocyclic monoterpenes, is used in mouth washes, soaps, toothpastes, and other cleansing formulations. Linalool, another well-known acyclic monoterpene alcohol, is among the commonly used ingredients added in perfumes and household cleaning agents. Due to its citrusy, fresh, and sweet odour, it is applied as a flavour enhancer in processed food and beverages. Citral, an acyclic monoterpene aldehyde, is the principle odourant of lemon oil. Owing to its citrusy and lemon-like smell that is strongly linked to the subjective impression of freshness and cleanliness, citral is also widely used in household products. Alpha- and beta-pinene, two bicyclic monoterpenes found in turpentine, are fragrance substances used to improve the odour of industrial products. Moreover, they are precursors of several flavour compounds such as citronellol, geraniol, menthol, and verbenol.

Although the use of essential oils has been primarily related to food flavourings, cosmetics, and perfumes due to their aroma, research demonstrates the high potential of the use of volatile monoterpene constituents to cure and prevent human diseases. Natural monoterpenes and their synthetic derivatives have repeatedly been described in relation to their various pharmacological effects: studies showed that monoterpenes have antimicrobial, anti-inflammatory, antispasmodic, analgesic, and anaesthetic effects. Moreover, in the last few years, several monoterpenes have been shown to be potential chemotherapeutic agents.

Among them, the monocyclic monoterpene limonene was found to inhibit the development of several types of cancer, such as liver, skin, and lung cancer. Geraniol, a monoterpene alcohol found in lemon grass, has been demonstrated to inhibit the growth of hepatoma, leukaemia, and colon cancer cells. Monoterpenes have also been studied in the prevention and treatment of cardiovascular diseases owing to their vasodilation and hypotension effects. Carvacrol, limonene, citronellol, myrtenol, and linalool are among these monoterpenes showing in-vivo and in-vitro cardiovascular effects in both humans and animals. Apart from that, other monoterpenoids, like thymol and terpineol, exerted potent antioxidant and free radical scavenging activities. Monoterpenes have also been shown to be anti-inflammatory and to positively impact several respiratory disorders. In particular, 1,8-cineol and menthol are among the most effective compounds for alleviating bronchopulmonary disorders. A recent study showed that both linalool and linalyl acetate, as anti-inflammatory compounds, were capable of decreasing the edema in the carrageenan-induced paw edema rat model following systemic administration.

As mentioned elsewhere herein, monoterpenes are effective antimicrobial agents. Specifically, some monoterpeneoids such as thymol, or menthol are effective against several gram-positive bacteria such as Staphylococcus aureus and gram-negative bacteria such as Escherichia coli. Other monoterpenes, namely geraniol and citral, exert antifungal activity against a human fungal pathogen called Cryptococcus neoformans, which usually infects lungs or the central nervous system. Moreover, citral is not only an antifungal agent but is also capable of reducing the infectivity of Herpes simplex virus (HSV), thus exihibiting a significant anti-HSV activity.

"Monoterpene alcohol" as used herein means a monoterpene (C10) comprising an alcohol group as a functional group. Monoterpene alcohols are well described in the art; see, e.g., DH Grayson. Monoterpenoids. Nat Prod Rep 1996 Jun;13(3):195-225. doi: 10.1039/np9961300195. Per definition, a primary alcohol is an alcohol in which the hydroxy group is bonded to a primary carbon atom. It can also be defined as a molecule containing a "-CH₂OH" group. In contrast, a secondary alcohol has a formula "-CHROH" and a tertiary alcohol has a formula "-CR₂OH", where "R" indicates a carbon-containing group.

Primary alcohols present in monoterpenes are referred to as "primary monoterpene alcohols", herein: The carbon to which the acceptor alcohol group is attached is bonded to one carbon group. Primary alcohols present in monoterpenes are, for example, geraniol, citronellol, and lavandulol.

Secondary alcohols present in monoterpenes are referred to as "secondary monoterpene alcohols", herein: The carbon to which the acceptor alcohol group is attached is bonded to two carbon groups. Secondary alcohols present in monoterpenes are, for instance, borneol, isoborneol, fenchol, verbenol, carveol, and menthol.

Tertiary alcohols present in monoterpenes are referred to as "tertiary monoterpene alcohols", herein: The carbon to which the acceptor alcohol group is attached is bonded to three carbon groups. Tertiary alcohols present in monoterpenes are, e.g., S-linalool, R-linalool, alpha terpineol, gamma terpineol, fenchol, p-cymene-8-ol, p-menth-3-en-1-ol, p-menth-8-en-1-ol, 4-carvomenthol, and 4-Thujanol; see, e.g., doi: 10.1111/1750-3841.12407.

Acyclic monoterpene alcohols, or monoterpenols as sometimes referred to in literature, are 2,6-dimethyloctane derivatives containing variable double bond moieties and a hydroxyl- function. The most important substances of this class are linalool, geraniol, nerol, citronellol, myrcenol, and dihydromyrcenol. They are used in perfumery because of their pleasant olfactory properties since ancient times.

Per definition, an ester is a chemical compound derived from an acid (organic or inorganic) in which at least one -OH (hydroxyl) group is replaced by an -O-alkyl (alkoxy) group.

"Monoterpene esters" as used herein means esters from monoterpene alcohols. The term includes esters from primary monoterpene alcohols, secondary monoterpene alcohols or tertiary monoterpene alcohols, as defined herein. Preferably, the term "monoterpene ester" as used herein is a "monoterpene acetyl ester". Unless indicated otherwise, said terms are used interchangeably herein.

"Sesquiterpene alcohol" as used herein means a sesquiterpene (C15) comprising an alcohol group as a functional group. Sesquiterpene alcohols are well known in the art; see, e.g., Fraga 2012, Natural sesquiterpenoids. Nat. Prod. Rep., 2013, 30, 1226.

The definitions of primary, secondary and tertiary alcohols in monoterpenes apply mutatis mutandis to sesquiterpene alcohols.

"Sesquiterpene esters" as used herein means esters from sesquiterpene alcohols. The term includes esters from primary sesquiterpene alcohols, secondary sesquiterpene alcohols or tertiary sesquiterpene alcohols, as defined herein.

In a preferred embodiment of the enzyme of the invention, the alcohol acyl transferase comprises an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 40% sequence identity at the amino acid level with SEQ ID NO: 2, or an amino acid sequence with at least 80% sequence identity at the amino acid level with SEQ ID NO: 3 (Lavandula AAT-10056) or SEQ ID NO: 4 (Lavandula AAT-1461); and
c) an enzymatically active fragment of the amino acid sequence of a) or b).

In a preferred embodiment, the enzyme of the invention is an enzymatically active alcohol acyl transferase comprising an amino acid sequence having at least 45%, at least 50%, at least 55%, at least 60%, at least 65%, at least 70%, at least 75%, at least 80%, at least 85%, at least 90%, at least 95%, at least 96%, at least 97%, at least 98%, at least 99 %, or even 100% sequence identity with SEQ ID NO: 2, at the amino acid level.

In a preferred embodiment, the enzyme of the invention is an enzymatically active alcohol acyl transferase comprising an amino acid sequence having at least 85%, at least 90%, at least 95%, at least 96%, at least 97%, at least 98%, at least 99 %, or even 100% sequence identity with SEQ ID NO: 3 or 4, at the amino acid level. Preferably, the amino acid sequence of this enzymatically active alcohol acyl transferase comprises the "MEIE" motif, with the consecutive amino acid residues methionine, glutamate, isoleucine, and glutamate.

Preferably, the sequence identity as referred to herein is determined over the full length of SEQ ID NO: 2, 3 and 4, respectively.

The amino acid sequences of the alcohol acyl transferases (AATs) of the invention are depicted in SEQ ID NO: 2, SEQ ID NO: 3 and SEQ ID NO: 4.

Specifically, SEQ ID NO: 2 corresponds to the amino acid sequence of the alcohol acyl transferase (AAT) (variant AAT9-1-c) from Citrus bergamia.

SEQ ID NO: 3 corresponds to the amino acid sequence of the alcohol acyl transferase (AAT) from Lavandula angustifolia (internal designation "10056").

SEQ ID NO: 4 corresponds to the amino acid sequence of the alcohol acyl transferase (AAT) from Lavandula angustifolia (internal designation "1461").

The cloning and functional characterization of these enzymes is shown in the following Examples.

Such alcohol acyl transferases with the indicated minimum percentage sequence identity have alcohol acyl transferase activity as defined herein and can be, for instance, homologues, variants, derivatives, or peptidomimentics, of the alcohol acyl transferases (AATs) of the invention. Methods for identification, cloning and functional characterization of such alcohol acyl transferases with the indicated minimum percentage sequence identity are known in the art, and described elsewhere herein as well as in the following Examples.

The term "protein" or "polypeptide" or "(poly)peptide" or "peptide" (all terms are used interchangeably, if not indicated otherwise) as used herein encompasses isolated and/or purified and/or recombinant (poly)peptides being essentially free of other host cell polypeptides.

The term "peptide" as referred to herein comprises at least two, three, four, five, six, seven, eight, nine, ten, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 60, 70, 80, 90, 100, 150, 200, 250, 300 or even more amino acid residues where the alpha carboxyl group of one is bound to the alpha amino group of another. A post-translational modification of the protein or peptide as used and envisaged herein is the modification of a newly formed protein or peptide and may involve deletion, substitution or addition of amino acids, chemical modification of certain amino acids, for example, amidation, acetylation, phosphorylation, glycosylation, formation of pyroglutamate, oxidation/reduction of sulfa group on a methionine, or addition of similar small molecules, to certain amino acids.

"Homologues" means bacterial, fungal, plant or animal homologues of the alcohol acyl transferases of the invention, preferably plant homologues, but also includes truncated sequences, single-stranded DNA or RNA of the coding and non-coding DNA sequence.

As demonstrated in Example 4, the present inventors were able to identify linalool acetyltransferase homologues in Lavandula angustifolia, the amino acid sequences of which are depicted in SEQ ID NO: 3 (Lavandula AAT-10056) and SEQ ID NO: 4 (Lavandula AAT-1461).

Sequence identity, homology or similarity is defined herein as a relationship between two or more amino acid sequences or two or more nucleic acid sequences, as determined by comparing those sequences. Usually, sequence identities or similarities are compared over the whole length of the sequences, but may also be compared only for a part of the sequences aligning with each other. Preferably, the sequence identities or similarities are compared over the whole length of the sequences, herein. In the art, "identity" or "similarity" also means the degree of sequence relatedness between polypeptide sequences or nucleic acid sequences, as the case may be, as determined by the match between such sequences.

Sequence alignments can be generated with a number of software tools, such as:
- Needleman and Wunsch algorithm - Needleman, Saul B. & Wunsch, Christian D. (1970). "A general method applicable to the search for similarities in the amino acid sequence of two proteins". Journal of Molecular Biology 48 (3): 443-453.

This algorithm is, for example, implemented into the "NEEDLE" program, which performs a global alignment of two sequences. The NEEDLE program, is contained within, for example, the European Molecular Biology Open Software Suite (EMBOSS).
- EMBOSS - a collection of various programs: The European Molecular Biology Open Software Suite (EMBOSS), Trends in Genetics 16 (6), 276 (2000).
- BLOSUM (BLOcks SUbstitution Matrix) - typically generated on the basis of alignments of conserved regions, e.g., of protein domains (Henikoff S, Henikoff JG: Amino acid substitution matrices from protein blocks. Proceedings of the National Academy of Sciences of the USA. 1992 Nov 15; 89(22): 10915-9). One out of the many BLOSUMs is "BLOSUM62", which is often the "default" setting for many programs, when aligning protein sequences.
- BLAST (Basic Local Alignment Search Tool) - consists of several individual programs (BlastP, BlastN) which are mainly used to search for similar sequence in large sequence databases. BLAST programs also create local alignments. Typically used is the "BLAST" interface provided by NCBI (National Center for Biotechnology Information), which is the improved version ("BLAST2"). The "original" BLAST: Altschul, S.F., Gish, W., Miller, W., Myers, E.W. & Lipman, D.J. (1990) "Basic local alignment search tool." J. Mol. Biol. 215:403-410; BLAST2: Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.

Sequence identity as used herein is preferably the value as determined by the EMBOSS Pairwise Alignment Algoritm "Needle". In particular, the NEEDLE program from the EMBOSS package can be used (version 2.8.0 or higher, EMBOSS: The European Molecular Biology Open Software Suite - Rice, P., et al. Trends in Genetics (2000) 16: 276-277; http://emboss.bioinformatics.nl) using the NOBRIEF option ('Brief identity and similarity' to NO) which calculates the "longest- identity". The identity, homology or similarity between the two aligned sequences is calculated as follows: Number of corresponding positions in the alignment showing an identical amino acid in both sequences divided by the total length of the alignment after subtraction of the total number of gaps in the alignment. For alignment of amino acid sequences the default parameters are: Matrix = Blosum62; Open Gap Penalty = 10.0; Gap Extension Penalty = 0.5. For alignment of nucleic acid sequences the default parameters are: Matrix = DNAfull; Open Gap Penalty = 10.0; Gap Extension Penalty = 0.5.

For instance, enzyme variants may be defined by their sequence identity when compared to a parent enzyme such as the alcohol acyl transferase of the invention with the amino acid sequence depicted in SEQ ID Nos. 2, 3, or 4. Sequence identity usually is provided as "% sequence identity" or "% identity". To determine the percent-identity between two amino acid sequences in a first step a pairwise sequence alignment is generated between those two sequences, wherein the two sequences are aligned over their complete, entire or full length (i.e., a pairwise global alignment). The alignment is generated with a program or software described herein. The preferred alignment for the purpose of this invention is that alignment, from which the highest sequence identity can be determined.

Discrepancies between an alcohol acyl transferase according to an amino acid sequence or a nucleic acid according to a specific sequence of the invention and a functional homologue of said enzyme or nucleic acid may in particular be the result of modifications performed, e.g., to improve a property of the enzyme or nucleic acid (e.g., improved expression of the enzyme or increased enzymatic activity of the enzyme) by a biological technique known to the skilled person in the art, such as, e.g., molecular evolution or rational design, or by using a mutagenesis technique known in the art and described elsewhere herein (random mutagenesis, site-directed mutagenesis, directed evolution, gene recombination, etc.).

The enzyme's or the nucleic acid's sequence may be altered, as a result of one or more natural occurring variations. Examples of such natural modifications or variations are differences in glycosylation (more broadly defined as "post-translational modifications"), differences due to alternative splicing, and single-nucleic acid polymorphisms (SNPs). The nucleic acid may be modified such that it encodes a polypeptide that differs by at least one amino acid, or two, three, four, five, six, or even more amino acids so that it encodes a polypeptide comprising one or more amino acid substitutions, deletions and/or insertions, which polypeptide still has alcohol acyl transferase activity, as defined herein. Further, use may be made of artificial gene-synthesis (synthetic DNA), codon optimisation or codon pair optimisation, e.g. based on a method as described in WO 2008/000632 or as offered by commercial DNA synthesizing companies like DNA2.0, Geneart, and GenScript.

The enzyme's or the nucleic acid's sequence may be altered by gene editing. Gene editing or genome editing is a type of genetic engineering in which DNA is inserted, replaced, or removed from a genome and which can be obtained by using a variety of techniques such as "gene shuffling" or "directed evolution" consisting of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547), or with "T-DNA activation" tagging (Hayashi et al. Science (1992) 1350-1353), where the resulting transgenic organisms show dominant phenotypes due to modified expression of genes close to the introduced promoter, or with "TILLING" (Targeted Induced Local Lesions In Genomes) and refers to a mutagenesis technology useful to generate and/or identify nucleic acids encoding proteins with modified expression and/or activity. TILLING also allows selection of organisms carrying such mutant variants. Methods for TILLING are well known in the art (McCallum et al., (2000) Nat Biotechnol 18: 455-457; reviewed by Stemple (2004) Nat Rev Genet 5(2): 145-50). Another technique uses artificially engineered nucleases like Zinc finger nucleases, Transcription Activator-Like Effector Nucleases (TALENs), the CRISPR/Cas system, and engineered meganuclease such as re-engineered homing endonucleases (Esvelt, KM.; Wang, HH. (2013), Mol Syst Biol 9 (1): 641; Tan, WS.et al. (2012), Adv Genet 80: 37-97; Puchta, H.; Fauser, F. (2013), Int. J. Dev. Biol 57: 629-637).

Derivatives of the alcohol acyl transferase of the invention comprise functional, i.e. enzymatically active variants which can be obtained by deletion, insertion, or substitution of amino acid residues from/into the amino acid sequence. A modification or mutation may be a replacement of an amino acid residue by a different one, a deletion of an amino acid residue, or an insertion of an amino acid residue. For instance, amino acid residues that are involved in substrate binding can be modified or mutated. The modified or mutated amino acid sequence has preferably improved, e.g., increased alcohol acyl transferase activity, in comparison to the wild type amino acid sequence of SEQ ID NO. 2, 3 or 4. To provide another example, the modified or mutated amino acid sequence can be an alcohol acyl transferase with an altered substrate specificity, for instance, in that it preferably esterifies a specific isomer of a monoterpene alcohol, or that the alcohol acyl transferase is able to esterify specific sesquiterpene alcohols, in addition to esterification of primary, secondary and/or tertiary monoterpene alcohols. To provide further examples, the modified or mutated amino acid sequence can be an alcohol acyl transferase which is capable of esterification of other non-terpene compounds, e.g. phenolic compounds, or capable of production of nor-compounds of terpenes, through acetylation of alcohols, e.g., coniferyl, or 2-phenyl ethanol.

DNA and the proteins that they encode can be modified using various techniques known in molecular biology to generate variant proteins or enzymes with new or altered properties (see, e.g., Sambrook; Ausubel, cited elsewhere herein).

Random PCR mutagenesis is described, e.g., in Rice (1992) Proc. Natl. Acad. Sci. USA 89:5467-5471, and combinatorial multiple cassette mutagenesis is described, e.g., in Crameri (1995) Biotechniques 18:194-196.

Alternatively, nucleic acids, e.g., genes, can be reassembled after random, or "stochastic," fragmentation, see, e.g., U.S. Patent Nos. 6,291,242; 6,287,862; 6,287,861; 5,955,358; 5,830,721; 5,824,514; 5,811,238; 5,605,793.

Alternatively, modifications, additions or deletions are introduced by error-prone PCR, shuffling, site-directed mutagenesis, assembly PCR, sexual PCR mutagenesis, in vivo mutagenesis (phage-assisted continuous evolution, in vivo continuous evolution), cassette mutagenesis, recursive ensemble mutagenesis, exponential ensemble mutagenesis, site-specific mutagenesis, gene reassembly, gene site saturation mutagenesis (GSSM), synthetic ligation reassembly (SLR), recombination, recursive sequence recombination, phosphothioate-modified DNA mutagenesis, uracil-containing template mutagenesis, gapped duplex mutagenesis, point mismatch repair mutagenesis, repair-deficient host strain mutagenesis, chemical mutagenesis, radiogenic mutagenesis, deletion mutagenesis, restriction-selection mutagenesis, restriction-purification mutagenesis, artificial gene synthesis, ensemble mutagenesis, chimeric nucleic acid multimer creation, and/or a combination of these and other methods.

Alternatively, "gene site saturation mutagenesis" or "GSSM" includes a method that uses degenerate oligonucleotide primers to introduce point mutations into a polynucleotide, as described in detail, in U.S. Patent Nos. 6,171,820 and 6,764,835.

Alternatively, Synthetic Ligation Reassembly (SLR) includes methods of ligating oligonucleotide building blocks together non-stochastically, as disclosed in, e.g., U.S. Patent No. 6,537,776.

Alternatively, Tailored multi-site combinatorial assembly ("TMSCA") is a method of producing a plurality of progeny polynucleotides having different combinations of various mutations at multiple sites by using at least two mutagenic non-overlapping oligonucleotide primers in a single reaction. Said method is described, e.g., in WO 2009/018449.

The term "protein" or "polypeptide" or "peptide" as used herein encompasses peptidomimetics of the alcohol acyl transferase of the invention. As known in the art, peptidomimetics are compounds whose essential elements (pharmacophore) mimic a natural peptide or protein in 3D space and which retain the ability to interact with the biological target (such as substrate of the enzyme) and produce the same biological effect (for example, alcohol acyl transferase activity); see, e.g., the review by Vagner et al. 2008, Current Opinion in Chemical Biology 12, Pages 292-296. Peptidomimetics are designed to circumvent some of the problems associated with a natural polypeptide, e.g., stability against proteolysis (duration of biological activity) and poor bioavailability. Certain other properties, such as selectivity for the biological target as mentioned above or potency of the biological activity, such as the aforementioned biological activity, often can be substantially improved.

Preferably, said homologues, variants, derivatives, or peptidomimetics of the alcohol acyl transferases (AATs) of the invention have at least 50%, 60%, 70%, 80%, 90%, or even 100% of the alcohol acyl transferase activity of the non-modified or non-mutated alcohol acyl transferase amino acid sequence of SEQ ID NO. 2, 3 or 4. To provide an example, the alcohol acyl transferase of the invention is capable of esterifying a tertiary monoterpene alcohol (e.g. linalool) such that at least 30% by mass of said tertiary monoterpene alcohol is esterified. A homologue, variant, derivative, or peptidomimetic of the alcohol acyl transferase (AATs) of the invention has 50% of the alcohol acyl transferase activity of the alcohol acyl transferase of the invention, if it is capable of esterifying said tertiary monoterpene alcohol such that at least 15% by mass of said tertiary monoterpene alcohol is esterified. Said homologues, variants, derivatives or peptidomimetics preferably also maintain the desired substrate specificity and/or substrate preference of the alcohol acyl transferase of SEQ ID NO. 2, 3 or 4. For instance, the homologue, variant, derivative or peptidomimetic is able to esterify primary, secondary and/or tertiary monoterpene alcohols, preferably tertiary monoterpene alcohols, as defined elsewhere herein.

To understand the molecular mechanism involved in ester biosynthesis by an alcohol acyl transferase of the invention and to clarify the importance of specific amino acid residues, structural models for the alcohol acyl transferase can be built by comparative modelling methodology. Afterwards, conformational interaction between the protein and several ligands, alcohols and acyl-CoAs can be explored by molecular docking and molecular dynamics simulation. Such methods are known and described in the art (e.g., Galaz et al., FEBS Journal 280 (2013) 1344-1357).

In a preferred embodiment, the alcohol acyl transferase of the invention is comprised by a fusion protein.

The alcohol acyl transferase of the invention can also be a fusion protein. The term "fusion protein" as used herein denotes a chimeric protein (literally, made of parts from different sources) which is created through the joining of two or more genes that originally coded for separate proteins. Translation of this fusion gene results in a single or multiple polypeptides with functional properties derived from each of the original proteins. For example, the fusion protein as defined herein can comprise an affinity tag for protein purification (such as His tag, FLAG tag etc., see, e.g. Kimple et al., 2015, Curr Protoc Protein Sci.; 73: Unit-9.9. doi:10.1002/0471140864.ps0909s73.), or a label for detection. A "label" as referred to herein is a detectable compound or composition that is conjugated directly or indirectly to another molecule, such as the alcohol acyl transferase of the invention, to facilitate detection of that molecule. Specific, non-limiting examples of labels include fluorescent tags, enzymatic linkages, and radioactive isotopes well known in the art. In one embodiment, a protease cleavage site and/or linker (i.e. a protease cleavage site; or a linker; or both a protease cleavage site and a linker; or the linker comprises a protease cleavage site) can be present between the the alcohol acyl transferase of the invention and label or purification tag. For instance, the protease cleavage site can be used to cleave off the purification tag by treatment with proteases, such as enterokinase or thrombin, if desired. For example, a His tag can be used as a tag for expression and purification while the alcohol acyl transferase of the invention can be isolated post-cleavage with the protease. As well known by the skilled person, besides the basic role in linking the functional domains together (as in flexible and rigid linkers), linkers may offer many other advantages for the production of fusion proteins, such as improving biological activity, increasing expression yield, and achieving desirable pharmacokinetic profiles. The linker can be, e.g., a protein/peptide linker such as a polyglycine linker or other linker known in the art (see, e.g., Chen et al., Adv Drug Deliv Rev. 2013; 65(10): 1357-1369). Evidently, the linker can be designed in a way that it comprises a protease cleavage site. In another aspect, the fusion protein can carry a signal peptide for targeting the expressed polypeptide, e.g. to a specific organelle, as explained elsewhere herein.

The fusion protein as defined herein can be manufactured by chemical synthesis or recombinant molecular biology techniques well known to the person skilled in the art. This applies mutatis mutandis to the isolation of fusion protein from the host cell or supernatant; see, e.g., Sambrook et al., Molecular cloning: a laboratory manual / Sambrook, Joseph; Russell, David W. --. 3rd ed. -- New York: Cold Spring Harbor Laboratory, 2001; Ausubel, Current Protocols in Molecular Biology, Green Publishing Associates and Wiley Interscience, N.Y. (1994).

The invention also relates to antibodies specifically binding to the alcohol acyl transferase of the invention.

As used herein, the term "antibody" refers to a molecule generated by an immune system that was made in response to a particular antigen that specifically binds to that antigen, and includes both naturally occurring antibodies and non-naturally occurring antibodies. An "antibody" as used herein encompasses a monoclonal antibody, a polyclonal antibody, a single chain antibody, a dimer or a multimer, a chimerized antibody, a bispecific antibody, a bispecific single chain antibody, a multispecific antibody, a synthetic antibody, a bifunctional antibody, a cell-associated antibody like an Ig receptor, a linear antibody, a diabody, a minibody, or a fragment of any of said antibodies, specifically binding to the alcohol acyl transferase of the invention. Fragments of said antibodies include, e.g., Fab, Fv, or scFv fragments, or chemically modified derivatives of any of these fragments. Antibodies can be manufactured by using methods which are described in the art; see, for example, Harlow and Lane "Antibodies, A Laboratory Manual", CSH Press, Cold Spring Harbor, 1988. Monoclonal antibodies can be prepared by the techniques originally described in Kohler 1975, Nature 256, 495, and Galfre 1981, Meth. Enzymol. 73, 3. Said techniques comprise the fusion of mouse myeloma cells to spleen cells derived from immunized mammals. The optical phenomenon of surface plasmon resonance (SPR) used by Biacore systems enables the detection and measurement of protein-protein interactions in real time, without the use of labels. For instance, the application of Biacore technology can be used to measure a protein-protein interaction using an antibody and its antigen, such as the alcohol acyl transferase of the invention, a fragment or an epitope thereof. The affinity of the antibody for its antigen is determined by measuring the binding kinetics of the interaction. Antibodies can be further improved by techniques well known in the art. Surface plasmon resonance as employed in the Biacore system can also be used to increase the efficiency of phage antibodies which bind to the epitope; see, e.g., Schier 1996, Human Antibodies Hybridomas 7, 97; Malmborg 1995, J. Immunol. Methods 183, 7.

The invention further pertains to a nucleic acid comprising a nucleic acid sequence encoding the alcohol acyl transferase of the invention, or a complementary sequence thereof. Preferably, the nucleic acid of the invention is suitable for encoding and producing an enzymatically active alcohol acyl transferase of the invention, in a microbial cell as defined herein.

The nucleic acid (or polynucleotide) of the invention comprises a nucleic acid sequence which encodes the alcohol acyl transferase of the invention. The nucleic acid sequence encoding the alcohol acyl transferase of the invention is preferably a recombinant and/or isolated and/or purified nucleic acid sequence. The nucleic acid sequence which encodes the alcohol acyl transferase of the invention can be produced and isolated using known molecular-biological standard techniques, the sequence information and organisms provided herein.

For example, a homologous sequence stretch or homologous, conserved sequence regions can be identified at the DNA or amino acid level, in the alcohol acyl transferase sequences of the invention and sequences of other vinorine synthase family members or vinorine synthase-like sequences (see Example 3), with the aid of comparative algorithms and sequence alignments. The identified sequences can then be used as hybridization probe in standard hybridization techniques, such as, for example, those described in Sambrook et al. (cited elsewhere herein) for cloning of the nucleic acid sequence encoding the alcohol acyl transferase of the invention. Alternatively, nucleic acid sequences encoding the alcohol acyl transferase of the invention can be produced by polymerase chain reaction well known in the art, using appropriate specific primers. The isolated or produced nucleic acid sequence can then be cloned into a suitable vector as described herein and characterized by means of DNA sequence analysis. The alcohol acyl transferase activity of the encoded protein can be determined after expression of the nucleic acid sequence in appropriate host cells, as described elsewhere herein and in Example 2.

For instance, the nucleic acid sequence of the invention can be isolated from Citrus bergamia or from Lavandula angustifolia.

Example 1 shows the cloning of the nucleic acid sequence encoding the alcohol acyl tranferase from Citrus bergamia. SEQ ID NO: 1 corresponds to the nucleotide sequence of the alcohol acyl transferase (AAT) from Citrus bergamia (variant AAT9-1-c). Further, Example 4 shows the identification of homologues of the alcohol acyl transferase (AAT) from Citrus bergamia in Lavandula angustifolia.

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full- length or a sub-sequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells. Every nucleic acid sequence herein that encodes a polypeptide such as the alcohol acyl transferase of the invention also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. The terms "polypeptide", "peptide" and "protein" are used interchangeably herein to refer to a polymer of amino acid residues. An " enzymatically active fragment of the amino acid sequence" of the alcohol acyl transferase of the invention means a stretch of at least 30, 35, 40, 45, 50, 60, 70, 80, 90, 100, 150, 200, or 250 amino acid residues having alcohol acyl transferase activity as defined herein. The terms "polypeptide", "peptide" and "protein" apply also to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulphation, gamma-carboxylation of glutamic acid residues, hydroxylation and ADP-ribosylation. Within the context of the present application, oligomers (such as oligonucleotides, oligopeptides) are considered a species of the group of polymers. Oligomers have a relatively low number of monomeric units, in general 2-100, in particular 6-100, including, e.g., primer sequences, such as used for cloning of the alcohol acyl transferase of the invention, in the Examples.

The term "heterologous" when used with respect to a nucleic acid (DNA or RNA) or protein of the invention refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins of the invention are not endogenous to the cell into which they are introduced, but have been obtained from another cell or synthetically or recombinantly produced. Generally, though not necessarily, such nucleic acids encode proteins that are not normally produced by the cell in which the DNA is expressed. A gene that is endogenous to a particular host cell but has been modified from its natural form, through, for example, the use of DNA shuffling, is also called heterologous. The term "heterologous" also includes non-naturally occurring multiple copies of a naturally occurring DNA sequence. Thus, the term "heterologous" may refer to a DNA segment that is foreign or heterologous to the cell, or homologous to the cell but in a position and/or a number within the host cell nucleic acid in which the segment is not ordinarily found. Exogenous DNA segments are expressed to yield exogenous polypeptides.

A "homologous" DNA sequence of the invention is a DNA sequence that is naturally associated with a host cell into which it is introduced. Any nucleic acid or protein that one of skill in the art would recognize as heterologous or foreign to the cell in which it is expressed is herein encompassed by the term heterologous nucleic acid or protein.

The terms "modified", "modification", "mutated", or "mutation", as used herein regarding proteins or polypeptides compared to another protein or polypeptide (in particular compared to the alcohol acyl transferase of the invention comprising or consisting of the amino acid sequences of SEQ ID NO. 2, 3, or 4) apply mutatis mutandis to nucleotide or nucleic acid sequences. The mentioned terms are used to indicate that the modified nucleotide or nucleic acid sequences encoding the protein or polypeptide having alcohol acyl transferase activity has at least one difference in the nucleotide or nucleic acid sequence compared to the nucleotide or nucleic acid sequence of the protein or polypeptide with which it is compared, e.g. the amino acid sequences of SEQ ID NO. 2, 3, or 4. The terms are used irrespective of whether the modified or mutated protein actually has been obtained by mutagenesis of nucleic acids encoding these amino acids or modification of the polypeptide or protein, or in another manner, e.g. using artificial gene-synthesis methodology. Mutagenesis is a well-known method in the art, and includes, for example, site-directed mutagenesis by means of PCR or via oligonucleotide-mediated mutagenesis, as described in Sambrook, J., and Russell, D.W. Molecular Cloning: A Laboratory Manual.3d ed., Cold Spring Harbor Laboratory Press, Cold Spring Harbor, NY, (2001). The term "modified", "modification", "mutated", or "mutation" as used herein regarding genes is used to indicate that at least one nucleotide in the nucleotide sequence of that gene or a regulatory sequence thereof, is different from the nucleotide sequence that it is compared with, e.g. a nucleotide sequence encoding the the amino acid sequences of SEQ ID NO. 2, 3, or 4. A modification or mutation may in a particular be a replacement of a nucleotide by a different one, a deletion of a nucleotide or an insertion of a nucleotide.

The invention also relates to a vector or gene construct comprising the nucleic acid of the invention.

Preferably, the vector or gene construct is suitable for encoding and producing an enzymatically active alcohol acyl transferase of the invention, in a microbial cell as defined herein.

The nucleic acid of the invention is operatively linked to expression control sequences allowing expression in prokaryotic or eukaryotic host cells, or isolated fractions thereof, in a vector or gene construct. Thus, in an aspect, the vector is an expression vector. Expression of the nucleic acid of the invention comprises transcription of the polynucleotide into a translatable mRNA. Regulatory elements ensuring expression in prokaryotic or eukaryotic host cells are well known in the art. In an aspect, they comprise regulatory sequences ensuring initiation of transcription and/or poly-A signals ensuring termination of transcription and stabilization of the transcript. Additional regulatory elements may include transcriptional as well as translational enhancers.

Possible regulatory elements permitting expression in prokaryotic host cells comprise, e.g., the lac-, trp- or tac- promoter in E. coli, or Rhodobacter promoters (https://doi.org/10.1073/pnas.2010Q87117), and examples for regulatory elements permitting expression in eukaryotic host cells are the AOX1- or the GAL1- promoter in yeast or the CMV-, SV40-, RSV-promoter (Rous sarcoma virus), CMV-enhancer, SV40-enhancer or a globin intron in mammalian and other animal cells. Plant promoters are described, e.g., in Plant Biotechnology: Principles and Applications, pp 117-172, 2017. Moreover, inducible expression control sequences may be used in an expression vector. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art, such as Okayama-Berg cDNA expression vector pcDV1 (Pharmacia), pBluescript (Stratagene), pCDM8, pRc/CMV, pcDNA1, pcDNA3 (Invitrogen) or pSPORT1 (Invitrogen). Expression vectors derived from viruses such as retroviruses, vaccinia virus, adeno-associated virus, herpes viruses, or bovine papilloma virus, may be used for delivery of the polynucleotide or vector into a targeted cell population.

Methods which are well known to those skilled in the art can be used to construct vectors or gene constructs comprising the nucleic acid of the invention; see, for example, the techniques described in Sambrook, Molecular Cloning A Laboratory Manual, Cold Spring Harbor Laboratory (2001) N.Y. and Ausubel, Current Protocols in Molecular Biology, Green Publishing Associates and Wiley Interscience, N.Y. (1994).

The term "gene" as used herein is used broadly to refer to any segment of nucleic acid associated with a biological function, such as the nucleic acid of the invention. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

The term "chimeric gene" as used herein refers to any gene that contains 1) DNA sequences, including regulatory and coding sequences that are not found together in nature, or 2) sequences encoding parts of proteins not naturally adjoined, or 3) parts of promoters that are not naturally adjoined. Accordingly, a chimeric gene may comprise regulatory sequences and coding sequences that are derived from different sources, or comprise regulatory sequences and coding sequences derived from the same source, but arranged in a manner different from that found in nature.

A "gene construct" as used herein can vary in complexity according to the insertion of interest. The construct can be designed to be inserted randomly into the genome of an organism, which is called transgenesis by addition, or can be designed to be inserted into the genome at a specific targeted site, into the correct position of a determined chromosome, which is called transgenesis by homologous recombination. In both cases, the construct must be impeccable, with structures to control gene expression, such as a promoter, a site of transcription initiation, a site of polyadenylation, and a site of transcription termination. That is, the information which is being inserted into the receptor genome has a beginning, middle, and an end, thus avoiding problems of uncontrolled expression in the host cell or organism.

The terms "open reading frame" and "ORF" as used herein refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

"Coding sequence" as used herein refers to a DNA or RNA sequence that codes for a specific amino acid sequence and excludes the non-coding sequences. It may constitute an "uninterrupted coding sequence", i.e. lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein.

"Regulatory sequences" as used herein refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences as well as sequences which may be a combination of synthetic and natural sequences. As is noted above, the term "suitable regulatory sequences" is not limited to promoters. Examples of regulatory sequences include promoters (such as transcriptional promoters, constitutive promoters, inducible promoters), operators, enhancers, mRNA ribosomal binding sites, and appropriate sequences which control transcription and translation initiation and termination. Nucleic acid sequences are "operably linked" when the regulatory sequence functionally relates to the DNA or cDNA sequence of the invention. As used herein, the term "operably linked" or "operatively linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame. Each of the regulatory sequences may independently be selected from heterologous and homologous regulatory sequences.

"Promoter" as used herein refers to a nucleotide sequence, usually upstream (5') to its coding sequence, which controls the expression of said coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

"Expression cassette" as used herein means a DNA sequence capable of directing expression of a particular nucleotide sequence, for example, a nucleotide sequence encoding the alcohol acyl transferase of the invention, in an appropriate host cell as defined herein, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example, antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development, e.g. in plant development.

The term "vector" as used herein refers to a construction comprised of genetic material designed to direct transformation of a targeted cell. A vector contains multiple genetic elements positionally and sequentially oriented, i.e., operatively linked with other necessary elements such that the nucleic acid in a nucleic acid cassette can be transcribed and when necessary, translated in the transformed cells. In particular, the vector may be selected from the group of viral vectors, (bacterio)phages, cosmids or plasmids. The vector may also be a yeast artificial chromosome (YAC), a bacterial artificial chromosome (BAC) or Agrobacterium binary vector. The vector may be in double or single stranded linear or circular form which may or may not be self transmissible or mobilizable, and which can transform host organisms such as, e.g., Rhodobacter either by integration into the cellular genome or exist extrachromosomally (e.g. autonomous replicating plasmid with an origin of replication). Specifically included are shuttle vectors by which is meant a DNA vehicle capable, naturally or by design, of replication in two different host organisms as defined herein. Preferably, the nucleic acid in the vector is under the control of, and operably linked to, an appropriate promoter or other regulatory elements for transcription in a host cell as specified herein. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell. Vectors containing a nucleic acid can be prepared based on methodology known in the art. For instance, use can be made of a cDNA sequence encoding the alcohol acyl transferase of the invention operably linked to suitable regulatory elements, such as transcriptional or translational regulatory nucleic acid sequences.

The term "vector" as used herein, includes reference to a vector for standard cloning work ("cloning vector") as well as to more specialized type of vectors, like an (autosomal) expression vector and a cloning vector used for integration into the chromosome of the host cell ("integration vector").

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector.

The term "expression vector" as used herein refers to a DNA molecule, linear or circular, that comprises a segment encoding a polypeptide of interest under the control of (i.e. operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular, an expression vector comprises a nucleotide sequence that comprises in the 5' to 3' direction and operably linked: (a) a transcription and translation initiation region that are recognized by the host organism, (b) a coding sequence for a polypeptide of interest, and (c) a transcription and translation termination region that are recognized by the host organism. "Plasmid" refers to autonomously replicating extrachromosomal DNA which is not integrated into a microorganism's genome and is usually circular in nature.

An "integration vector" refers to a DNA molecule, linear or circular, that can be incorporated, e.g., into a microorganism's genome, such as a bacteria's genome, and provides for stable inheritance of a gene encoding a polypeptide of interest, such as the alcohol acyl transferase of the invention. The integration vector generally comprises one or more segments comprising a gene sequence encoding a polypeptide of interest under the control of (i.e., operably linked to) additional nucleic acid segments that provide for its transcription.

Such additional segments may include promoter and terminator sequences, and one or more segments that drive the incorporation of the gene of interest into the genome of the target cell, usually by the process of homologous recombination. Typically, the integration vector will be one which can be transferred into the target cell, but which has a replicon which is non-functional in that organism. Integration of the segment comprising the gene of interest may be selected if an appropriate marker is included within that segment. One or more nucleic acid sequences encoding appropriate signal peptides that are not naturally associated with a polypeptide to be expressed in a host cell of the invention can be incorporated into (expression) vectors. For example, a DNA sequence for a signal peptide leader can be fused in-frame to a nucleic acid of the invention so that the alcohol acyl transferase of the invention is initially translated as a fusion protein comprising the signal peptide. Depending on the nature of the signal peptide, the expressed polypeptide will be targeted differently. A secretory signal peptide that is functional in the intended host cells, for instance, enhances extracellular secretion of the expressed polypeptide. Other signal peptides direct the expressed polypeptide to certain organelles, like the chloroplasts, mitochondria and peroxisomes. The signal peptide can be cleaved from the polypeptide upon transportation to the intended organelle or from the cell. It is possible to provide a fusion of an additional peptide sequence at the amino or carboxyl terminal end of the polypeptide.

In addition, the invention concerns a host cell comprising the vector or gene construct of the invention.

The host cell is transformed with the the vector or gene construct of the invention. The skilled artisan is well aware of the genetic elements that must be present on the genetic construct to successfully transform, select and propagate host cells containing the vector or gene construct of the invention. The host cell of the invention is capable of expressing a polypeptide with alcohol acyl transferase activity, included in the vector or gene construct of the invention. "Transformation" and "transforming", as used herein, refers to the introduction of a heterologous nucleotide sequence, such as the nucleotide sequence encoding the alcohol acyl transferase of the invention, into a host cell, irrespective of the method used for the insertion, for example, direct uptake, transduction, conjugation, f-mating or electroporation. The exogenous polynucleotide may be maintained as a non-integrated vector, for example, a plasmid, or alternatively, may be integrated into the host cell genome.

A host cell according to the invention may be produced based on standard genetic and molecular biology techniques that are generally known in the art, e.g., as described in Sambrook, J., and Russell, D.W. "Molecular Cloning: A Laboratory Manual" 3d ed., Cold Spring Harbor Laboratory Press, Cold Spring Harbor, NY, (2001); and F.M. Ausubel et al, eds., "Current protocols in molecular biology", John Wiley and Sons, Inc., New York (1987), and later supplements thereto.

The host cell can be any cell selected from a microbial cell, e.g., a bacterial cell, a archaeal cell, a fungal cell, such as a yeast cell, and a protist cell. The host cell can also be an algal cell or a cyanobacterial cell, a non-human animal cell or a mammalian cell, or a plant cell.

Specifically, the host cell can be selected from any one of the following organisms:

### Bacteria

The bacterial host cell can, for example, be selected from the group consisting of the genera Escherichia, Klebsiella, Helicobacter, Bacillus, Lactobacillus, Streptococcus, Amycolatopsis, Rhodobacter, Pseudomonas, Paracoccus or Lactococcus.

### gram positive: Bacillus, Streptomyces

Useful gram positive bacterial host cells include, but are not limited to, a Bacillus cell, e.g., Bacillus alkalophius, Bacillus amyloliquefaciens, Bacillus brevis, Bacillus circulans, Bacillus clausii, Bacillus coagulans, Bacillus firmus, Bacillus Jautus, Bacillus lentus, Bacillus licheniformis, Bacillus megaterium, Bacillus pumilus, Bacillus stearothermophilus, Bacillus subtilis, and Bacillus thuringiensis. Most preferred, the prokaryote is a Bacillus cell, preferably, a Bacillus cell of Bacillus subtilis, Bacillus pumilus, Bacillus licheniformis, or Bacillus lentus.

Some other preferred bacteria include strains of the order Actinomycetales, preferably, Streptomyces, preferably Streptomyces spheroides (ATTC 23965), Streptomyces thermoviolaceus (IFO 12382), Streptomyces lividans or Streptomyces murinus or Streptoverticillum verticillium ssp. verticillium. Other preferred bacteria include Rhodobacter sphaeroides, Rhodomonas palustri, Streptococcus lactis. Further preferred bacteria include strains belonging to Myxococcus, e.g., M. virescens.

### gram negative: E. coli, Pseudomonas, Rhodobacter, Paracoccus

Preferred gram negative bacteria are Escherichia coli, Pseudomonas sp., preferably, Pseudomonas purrocinia (ATCC 15958) or Pseudomonas fluorescens (NRRL B-11), Rhodobacter capsulatus or Rhodobacter sphaeroides, Paracoccus carotinifaciens or Paracoccus zeaxanthinifaciens).

### Fungi

### Aspergillus, Fusarium, Trichoderma

The host cell may be a fungal cell. "Fungi" as used herein includes the phyla Ascomycota, Basidiomycota, Chytridiomycota, and Zygomycota as well as the Oomycota and Deuteromycotina and all mitosporic fungi. Representative groups of Ascomycota include, e.g., Neurospora, Eupenicillium (=Penicillium), Emericella (=Aspergillus), Eurotium (=Aspergillus), and the true yeasts listed below. Examples of Basidiomycota include mushrooms, rusts, and smuts. Representative groups of Chytridiomycota include, e.g., Allomyces, Blastocladiella, Coelomomyces, and aquatic fungi. Representative groups of Oomycota include, e.g. Saprolegniomycetous aquatic fungi (water molds) such as Achlya. Examples of mitosporic fungi include Aspergillus, Penicillium, Candida, and Alternaria. Representative groups of Zygomycota include, e.g., Rhizopus and Mucor.

Some preferred fungi include strains belonging to the subdivision Deuteromycotina, class Hyphomycetes, e.g., Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli or Dreschlera halodes.
Other preferred fungi include strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Trametes (previously called Polyporus), e.g. T. versicolor (e.g. PR4 28-A).
Further preferred fungi include strains belonging to the subdivision Zygomycotina, class Mycoraceae, e.g. Rhizopus or Mucor, in particular Mucor hiemalis.

### Yeast

### Pichia

### Saccharomyces

The fungal host cell may be a yeast cell. Yeast as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (e.g., genus Schizosaccharomyces), Nadsonioideae, Lipomycoideae, and Saccharomycoideae (e.g. genera Kluyveromyces, Pichia, and Saccharomyces). The basidiosporogenous yeasts include the genera Leucosporidim, Rhodosporidium, Sporidiobolus, Filobasidium, and Filobasidiella. Yeasts belonging to the Fungi Imperfecti are divided into two families, Sporobolomycetaceae (e.g., genera Sporobolomyces and Bullera) and Cryptococcaceae (e.g. genus Candida).

### Eukaryotes

Eukaryotic host cells further include, without limitation, a non-human animal cell, a non-human mammal cell, an avian cell, reptilian cell, insect cell, or a plant cell.

In a preferred embodiment, the host cell is a host cell selected from:
a) a bacterial cell of the group of Gram negative bacteria, such as Rhodobacter (e.g. Rhodobacter sphaeroides or Rhodobacter capsulatus), Paracoccus (e.g. P. carotinifaciens, P. zeaxanthinifaciens), Escherichia or Pseudomonas;
b) a bacterial cell selected from the group of Gram positive bacteria, such as Bacillus, Corynebacterium, Brevibacterium, Amycolatopis;
c) a fungal cell selected from the group of Aspergillus, Blakeslea, Peniciliium, Phaffia (Xanthophyllomyces), Pichia, Saccharamoyces, Kluyveromyces, Yarrowia, and Hansenula;
d) a transgenic plant cell or a culture comprising transgenic plant cells, wherein the cell is of a transgenic plant selected from Arabidopsis spp., Nicotiana spp, Cichorum intybus, lacuca sativa, Mentha spp, Artemisia annua, tuber forming plants, oil crops, e.g. Brassica spp. or Brassica napus, flowering plants (angiosperms) which produce fruits, and trees;
or e) a transgenic mushroom or culture comprising transgenic mushroom cells, wherein the microorganism is selected from Schizophyllum, Agaricus and Pleurotisi.

More preferred host cells from organisms are host cells from microorganisms belonging to the genus Escherichia, Saccharomyces, Pichia, Rhodobacter, Pseudomonas or Paracoccus, (e.g. Paracoccus carotinifaciens, Paracoccus zeaxanthinifaciens) and even more preferred those of the species E.coli, S. cerevisae, Rhodobacter sphaeroides, Rhodobacter capsulatus, or Amycolatopis sp.

Particularly preferred is a Rhodobacter host cell selected from the group of Rhodobacter capsulatus and Rhodobacter sphaeroides.

In one preferred embodiment, the host cell is used for the production of the alcohol acyl transferase of the invention.

The method for producing the alcohol acyl transferase of the invention, preferably comprises the following steps: (a) culturing a host cell comprising a nucleic acid sequence encoding the alcohol acyl transferase of the invention, under appropriate conditions, and (b) obtaining from the host cell of step (a) the alcohol acyl transferase protein of the invention.
In another preferred embodiment, the host cell is suitable for carrying out the methods of the invention.

For instance, the host cell can be used in a method for preparing a monoterpene ester, comprising esterifying a monoterpene alcohol to a monoterpene ester, in the presence of the alcohol acyl transferase of the invention. To this end, the host cell preferably heterologously expresses the alcohol acyl transferase of the invention. It is preferred that the monoterpene alcohol is linalool, geraniol, alpha terpineol, gamma terpineol, lavandulol, fenchol, perillyl alcohol, menthol or verbenol, is used as substrate. The monoterpene alcohol substrate can be produced by the host cell or added exogenously to the host cell. The produced monoterpene ester is preferably a monoterpene ester or acetyl ester selected from the group consisting of linalyl acetate, geranyl acetate, alpha-terpineol ester (alpha-terpinyl acetate), gamma-terpineol ester (gamma-terpinyl acetate), the ester of lavandulol, the ester of fenchol, the ester of perillyl alcohol, the ester of menthol and the ester of verbenol, as set forth elsewhere herein.

To provide a specific example, the host cell can be used in a method for preparing linalyl acetate, comprising esterifying linalool to linalyl acetate, in the presence of the alcohol acyl transferase of the invention. The host cell contains or produces linalool as substrate, in this case.

In still another preferred embodiment, the host cell according to the invention can be used industrially in the fermentative production of a monoterpene ester as exemplified above. Such fermentative production is set forth elsewhere herein.

Preferably, the alcohol acyl transferase of the invention can also be used, in combination with one or more further enzymes, in the host cell of the invention.

One example for such an additional enzyme that can be used in addition to the alcohol acyl transferase of the invention, is linalool synthase. Linalool synthase is an enzyme that uses geranyl pyrophosphate as a substrate and catalyzes the formation of linalool. For instance, S-Linalool synthase stereoselectively converts the ubiquitous C10 intermediate Geranyl diphosphate (GPP) to S-linalool (Pichersky et al., Arch Biochem Biophys 1995 Feb 1;316(2):803-7. doi: 10.1006/abbi.1995.1107.). R-Linalool synthase specifically catalyzes the production of R-linalool. S- or R-Linalool is then esterified to linalyl acetate, by the alcohol acyl transferase of the invention. In this case, the host cell contains or produces geranyl pyrophosphate (GPP) as substrate for the linalool synthase.

Although linalool synthase employs one catalytic mechanism (exemplified by limonene synthase (LMS), it has sequence motifs indicative of both LMS-type synthases and the terpene synthases employing a different mechanism (exemplified by copalyl diphosphate synthase (CPS)), suggesting that linalool synthase appears to be a composite gene which might have evolved from a recombination event between two different types of terpene synthases (Cseke et al., Mol. Biol. Evol. 15(11):1491-1498. 1998).

The linalool synthase can be an R-linalool synthase or an S-linalool synthase.

R-linalool synthase (EC 4.2.3.26) catalyses the reaction geranyl diphosphate + H2O = (3S)-linalool + diphosphate.

S-linalool synthase (EC 4.2.3.25) catalyses the reaction geranyl diphosphate + H2O = (3R)-linalool + diphosphate.

Linalool synthases and their corresponding sequences are well known in the art.

For instance, UniProt Q2XSC5 shows the amino acid sequence of the R-linalool synthase from Lavandula angustifolia (Lavender). UniProt Q8H2B4 shows the amino acid sequence of the R-linalool synthase from Mentha aquatic. UniProt Q84UV0 shows the amino acid sequence of the S-linalool synthase from Arabidopsis thaliana.

Further accession numbers for linalool synthases, the species and the reference are indicated below.

### R-linalool synthase:

UniProt Q8H2B4 from Mentha aquatica: "Molecular cloning and characterization of a new linalool synthase." Crowell A.L., Williams D.C., Davis E.M., Wildung M.R., Croteau R. Arch. Biochem. Biophys. 405:112-121 (2002).

UniProt Q2XSC5 from Lavandula angustifolia "Cloning and functional characterization of three terpene synthases from lavender (Lavandula angustifolia)." Landmann C., Fink B., Festner M., Dregus M., Engel K.H., Schwab W. Arch. Biochem. Biophys. 465:417-429 (2007).

UniProt Q9SPN0; J W Jia 1 , J Crock, S Lu, R Croteau, X Y Chen. "(3R)-Linalool synthase from Artemisia annua L.: cDNA isolation, characterization, and wound induction." Arch Biochem Biophys. 1999 Dec 1;372(1):143-9. doi: 10.1006/abbi.1999.1466.

### S-linalool synthase:

UniProt Q6ZH94; "Jasmonate induction of the monoterpene linalool confers resistance to rice bacterial blight and its biosynthesis is regulated by JAZ protein in rice." Taniguchi S., Hosokawa-Shinonaga Y., Tamaoki D., Yamada S., Akimitsu K., Gomi K.
Plant Cell Environ. 37:451-461 (2014).

GenBank AFK09263; "Characterization of S-(+)-linalool synthase from several provenances of Cinnamomum osmophloeum." Yan-Liang Lin, Yi-Ru Lee, Wen-Ke Huang, Shang-Tzen Chang & Fang-Hua Chu Tree Genetics & Genomes volume 10, pages 75-86 (2014).

UniProt Q96376; N Dudareva, L Cseke, V M Blanc, E Pichersky. "Evolution of floral scent in Clarkia: novel patterns of S-linalool synthase gene expression in the C. breweri flower." The Plant Cell Jul 1996, 8 (7) 1137-1148; DOI: 10.1105/tpc.8.7.1137.

In another aspect, the host cell can comprise a Geranyl diphosphate (GPP) synthase, in addition to the alcohol acyl transferase of the invention and a linalool synthase. Geranyl diphosphate synthase catalyzes the condensation of dimethylallyl diphosphate and isopentenyl diphosphate to geranyl diphosphate (GPP). A Linalool synthase then converts the ubiquitous C10 intermediate GPP to linalool, which in turn is esterified to linalyl acetate, by the alcohol acyl transferase of the invention (Burke et al., Proc Natl Acad Sci USA. 1999 Nov 9; 96(23): 13062-13067). Evidently, the host cell contains or produces the substrates for the Geranyl diphosphate synthase, in this case.

Non-limiting examples for accession numbers for GPP synthases and the species are indicated below:
GenBank CAC16849.1 geranyl diphosphate synthase (Arabidopsis thaliana).
GenBank CAC16851.1 geranyl diphosphate synthase (Citrus sinensis).
GenBank ACA21458.2 geranyl diphosphate synthase 2 (Picea abies).

Moreover, the host cell of the invention is suitable for carrying out the uses of the invention as indicated herein.

Furthermore, the invention pertains to a transgenic non-human organism comprising the nucleic acid of the invention, the vector or gene construct of the invention, or the host cell of the invention.

The transgenic non-human organism of the invention comprises the nucleic acid of the invention, the vector or gene construct of the invention, or the host cell of the invention. In a preferred embodiment, the transgenic non-human organism of the invention is used for preparing a monoterpene ester as defined herein such as - but not limited to - linalyl acetate, as described in more detail elsewhere in this specification. The monoterpene ester is prepared by the action of the alcohol acyl transferase of the invention which esterifies the corresponding monoterpene alcohol. Said corresponding monoterpene alcohol is linalool, in the case of linalyl acetate.

Preferably, the transgenic non-human organism of the invention is a bacteria, a yeast, a fungus, a protist, an algae or a cyanobacteria, a non-human animal or a non-human mammalian, or a plant. Specifically, the organisms mentioned in connection with host cells of the invention can also be used for the generation of a transgenic non-human organism of the present invention.

It is preferred that the bacteria is a Gram negative bacteria, preferably Rhodobacter, Escherichia, Pseudomonas or Paracoccus.

The term "transgenic" for a transgenic organism or transgenic cell as used herein, refers to an organism or cell (which cell may be an organism per se or a cell of a multi-cellular organism from which it has been isolated) containing a nucleic acid not naturally occurring in that organism or cell and which nucleic acid has been introduced into that organism or cell (i.e., has been introduced in the organism or cell itself or in an ancestor of the organism or an ancestral organism of an organism of which the cell has been isolated) using recombinant DNA techniques known in the art. Or to put it differently: The nucleic acid is heterologous to that transgenic organism or transgenic cell.

A "transgene" refers to a gene such as an alcohol acyl transferase gene that has been introduced into the genome by transformation and preferably is stably maintained. Preferably, transgenes include genes that are heterologous to the genes of a particular cell or organism to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

Methods for the production of transgenic non-human organisms are well known in the art; see, e.g. Lee-Yoon Low et al., Transgenic Plants: Gene constructs, vector and transformation method. 2018. DOI.10.5772/intechopen.79369; Pinkert, C. A. (ed.) 1994. Transgenic animal technology: A laboratory handbook. Academic Press, Inc., San Diedo, Calif.; Monastersky G. M. and Robl, J. M. (ed.) (1995) Strategies in Transgenic Animal Science. ASM Press. Washington D.C); Sambrook, loc.cit, Ausubel, loc.cit).

The invention further relates to a method for preparing a monoterpene ester, comprising esterifying a monoterpene alcohol to a monoterpene ester, in the presence of an alcohol acyl transferase of the invention.

In this method of the invention, the alcohol acyl transferase of the invention is used for the production of a monoterpene ester by esterification of a monoterpene alcohol to its corresponding monoterpene ester.

In principle, the production of the monoterpene esters can be carried out in a manner based on methodology known in the prior art; see, e.g., Jongedijk et al., Appl Microbiol Biotechnol (2016) 100:2927-2938; Yee et al., Metab Eng. 2019 Sep;55:76-84. doi: 10.1016/j.ymben.2019.06.004; Fromighieri et al., Planta. 2018 Oct;248(4):933-946. doi: 10.1007/s00425-018-2948-0; Leferink et al., Sci Rep. 2019 Aug 15;9(1):11936. doi: 10.1038/s41598-019-48452-2; Zhao et al., Microb Cell Fact (2017) 16:17 DOI 10.1186/s12934-017-0641-9; or WO 2014/014339.

As substrates, the following monoterpene alcohols can be used:
The monoterpene alcohol can be a primary, secondary or tertiary monoterpene alcohol, such as linalool, geraniol, alpha terpineol, gamma terpineol, lavandulol, fenchol, perillyl alcohol, menthol or verbenol.

Preferably, the monoterpene alcohol is linalool, geraniol, alpha terpineol, gamma terpineol, lavandulol, fenchol, perillyl alcohol, menthol or verbenol.

The monoterpene alcohol is more preferably a tertiary monoterpene alcohol, for example, linalool or alpha-terpineol.

The monoterpene ester products prepared in this method of the invention can be: linalyl acetate, geranyl acetate, alpha-terpineol ester (alpha-terpinyl acetate), gamma-terpineol ester (gamma-terpinyl acetate), the ester of lavandulol, the ester of fenchol, the ester of perillyl alcohol, the ester of menthol or the ester of verbenol.

In a preferred embodiment, the method for preparing a monoterpene ester, comprises esterifying a monoterpene alcohol to a monoterpene ester, in the presence of an alcohol acyl transferase which is capable of esterifying a tertiary monoterpene alcohol such that at least 30% by mass of said tertiary monoterpene alcohol is esterified, preferably within 36 h, 24 h, 12 h, 6 h, 3 h, 2 h, 1h, 45 min or 30 min, preferably in a microbial cell as defined herein.

More preferably, the alcohol acyl transferase comprises an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 30%, preferably 40%, 45%, 50%, 55%, 60%, 65%, 70%, 75%, 80%, 85%, 90% or 95% sequence identity at the amino acid level with SEQ ID NO: 2, SEQ ID NO: 3 (Lavandula AAT-10056), or SEQ ID NO: 4 (Lavandula AAT-1461); and
c) an enzymatically active fragment of the amino acid sequence of a) or b),
in the method for preparing a monoterpene ester, of the invention.

Preferably, an amino acid sequence with at least 30%, preferably 40%, 45%, 50%, 55%, 60%, 65%, 70%, 75%, 80%, 85%, 90% or 95% sequence identity at the amino acid level with SEQ ID NO: 3 (Lavandula AAT-10056) or SEQ ID NO: 4 (Lavandula AAT-1461) comprises the "MEIE" motif, with the consecutive amino acid residues methionine, glutamate, isoleucine, and glutamate.

Even more preferably, the alcohol acyl transferase comprises an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 40% sequence identity at the amino acid level with SEQ ID NO: 2, or an amino acid sequence with at least 80% sequence identity, or preferably 85% sequence identity at the amino acid level with SEQ ID NO: 3 (Lavandula AAT-10056) or SEQ ID NO: 4 (Lavandula AAT-1461); and
c) an enzymatically active fragment of the amino acid sequence of a) or b),
in the method for preparing a monoterpene ester, of the invention.

In another preferred embodiment of this method of the invention, the monoterpene ester is prepared in a host cell or a non-human transgenic organism, expressing the alcohol acyl transferase of the invention.

In another embodiment, the host cell according to the invention can be used industrially in the fermentative production of the aforementioned monoterpene esters.

For instance, the host cell or non-human transgenic organism of the invention can be used in a fermentative production of monoterpene esters.

Preferably, the monoterpene ester is produced in a fermentative process, i.e. in a method comprising cultivating, e.g., microbial host cells such as Rhodobacter host cells in a culture medium under conditions wherein the alcohol acyl transferase of the invention is expressed.

The actual reaction catalysed by the alcohol acyl transferase of the invention typically takes place intracellularly. It should be noted that the term "fermentative" is used herein in a broad sense for processes wherein use is made of a culture of an organism to synthesize a compound from a suitable feedstock (e.g. a carbohydrate, an amino acid source, a fatty acid source). Thus, fermentative processes as meant herein are not limited to anaerobic conditions, and extended to processes under aerobic conditions. Suitable feedstocks are generally known for Rhodobacter host cells. Suitable conditions can be based on known methodology for Rhodobacter host cells, e.g. described in WO 2011/074954 or in WO 2014/014339.

The prepared monoterpene esters can be isolated or extracted from the host cell or non-human transgenic organism by methods known in the art (for plants, see, e.g., Jiang et al., Curr Protoc Plant Biol. 2016; 1: 345-358. doi:10.1002/cppb.20024; for Rhodobacter, see, e.g., WO 2014/014339).

In general, the methods include the following steps: 1) breaking the cells to release their chemical constituents; 2) extracting the sample using a suitable solvent (or through distillation or the trapping of compounds); 3) separating the desired monoterpene ester from other undesired contents of the extracts that confound analysis and quantification; and 4) use an appropriate method of analysis (e.g., thin layer chromatography (TLC), gas chromatography (GC), or liquid chromatography (LC) or another method as described herein).

Preferably, the alcohol acyl transferase of the invention is used in combination with a linalool synthase, and/or a Geranyl diphosphate (GPP) synthase, in this method of the invention. Linalool synthase and Geranyl diphosphate (GPP) synthase have already been described herein, in connection which host cells. The definitions, sequences and explanations with respect to the latter enzymes apply mutatis mutandis to the methods of the invention.

The monoterpene ester produced in accordance with this method of the invention may be used as such, e.g. as a flavour or fragrance, as an insect repellent, as a pesticide, or as an antimicrobial; it can also be used for producing biofuel, a fuel composition or a fuel compound such as - but not limited to - a blowing agent for diesel fuel compositions, or may be used as a starting material for another compound, e.g. another flavour or fragrance or fuel compound.

The invention also relates to a method for preparing linalyl acetate, comprising esterifying linalool to linalyl acetate, in the presence of an alcohol acyl transferase of the invention.

In this method, the alcohol acyl transferase of the invention is used for the production of linalyl acetate by esterification of linalool to its corresponding monoterpene ester, linalyl acetate.

In a preferred embodiment, the method for preparing linalyl acetate, comprises esterifying linalool to linalyl acetate, in the presence of an alcohol acyl transferase which is capable of esterifying linalool such that at least 30% by mass of linalool is esterified, preferably within 36 h, 24 h, 12 h, 6 h, 3 h, 2 h, 1h, 45 min or 30 min, preferably in a microbial cell as defined herein.

More preferably, the alcohol acyl transferase comprises an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 30%, preferably 40%, 45%, 50%, 55%, 60%, 65%, 70%, 75%, 80%, 85%, 90% or 95% sequence identity at the amino acid level with SEQ ID NO: 2, SEQ ID NO: 3 (Lavandula AAT-10056) or SEQ ID NO: 4 (Lavandula AAT-1461); and
c) an enzymatically active fragment of the amino acid sequence of a) or b),
in the method for preparing linalyl acetate, of the invention.

Preferably, an amino acid sequence with at least 30%, preferably 40%, 45%, 50%, 55%, 60%, 65%, 70%, 75%, 80%, 85%, 90% or 95% sequence identity at the amino acid level with SEQ ID NO: 3 (Lavandula AAT-10056) or SEQ ID NO: 4 (Lavandula AAT-1461) comprises the "MEIE" motif, with the consecutive amino acid residues methionine, glutamate, isoleucine, and glutamate.

Even more preferably, the alcohol acyl transferase comprises an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 40% sequence identity at the amino acid level with SEQ ID NO: 2, or an amino acid sequence with at least 80% sequence identity, or preferably 85% sequence identity at the amino acid level with SEQ ID NO: 3 (Lavandula AAT-10056) or SEQ ID NO: 4 (Lavandula AAT-1461); and
c) an enzymatically active fragment of the amino acid sequence of a) or b),
in the method for preparing linalyl acetate, of the invention.

In another preferred embodiment of this method of the invention, the linalyl acetate is prepared in a host cell or a non-human transgenic organism, expressing the alcohol acyl transferase of the invention.

Linalool, or 3,7-dimethyl-1,6-octadien-3-ol, is an acyclic, unsaturated, tertiary monoterpene alcohol, comprising two enantiomers that are both found in plants: (3S)-(+)- linalool and (3R)-(-)-linalool. Thereby, the latter is more common in nature. It is one of the main ingredients of essential oils of over 200 plant species belonging to over 50% of plant families. Among the most well-known plants that are rich in linalool are rosewood (Aniba rosaeodora Ducke, Lauraceae), Ho leaf (Cinnamomum camphora Nees, Lauraceae), fruits of coriander (Coriandrumsativum L., Apiaceae), and flowering tops of lavender (Lavendula officinalis Chaix sin., L.angustifolia Mill., Lamiaceae).

As already explained in the introductory part, linalyl acetate, the monoterpene ester of linalool, is an important flavour and fragrance molecule. Linalyl acetate is used in many fragrances, including amber, bubble gum, ginger, lavender, orange, rose, sandalwood, sherry, tuberose, ylang. Notably, it is an important component of perfume ingredients lavender oil and bergamot oil. In flavours, it is used for tomato, tropical and earl-grey tea flavourings.

Linalyl acetate occurs in two optical forms: the R-form (CAS No. 115-95-7) and the S-form (CAS No. 51685-40-6).

In bergamot, lavender, *Mentha citrata* (Bergamot mint) and a variety of other plants, there is (R)-linalyl acetate.

In cardamom oil, there is (S)-linalyl acetate.

Natural linalyl acetate, which is in most cases in the R-form, has been described to have a sweet, green, citrus, spicy odour, and a floral, green, terpy, waxy, citrus, woody taste.

Most linalyl acetate currently on the market is made by synthetic chemistry. This linalyl acetate is a mixture of both S- and R-isomers.

Enantiopure R-linalyl acetate is extracted from a number of sources, including bergamot oil, petitgrain (a steam distillate from bitter orange plant material, including leaves and twigs), neroli bigarat oil and bergamot mint oil. It is also known to be available from Ho wood oil (Cinnamomum camphora) from China, but there is pressure due to adulteration.

Preferably, the linalyl acetate prepared by the method of the invention is (i) in the R-form (Cas No. 115-95-7), (ii) in the S-form (Cas No. 51685-40-6), or (iii) in the R-form (Cas No. 115-95-7) and in the S-form (Cas No. 51685-40-6). The latter embodiment (iii) comprises a mixture of the R-form and the S-form of linalyl acetate.

Preferably, the alcohol acyl transferase of the invention is used in combination with a linalool synthase, and/or a Geranyl diphosphate (GPP) synthase, in this method of the invention. Linalool synthase and Geranyl diphosphate (GPP) synthase have already been described herein, in connection which host cells. The definitions, sequences and explanations with respect to the latter enzymes apply mutatis mutandis to the methods of the invention.

The invention also concerns the use of the alcohol acyl transferase of the invention, the nucleic acid of the invention, the vector or gene construct of the invention, the host cell of the invention, or the transgenic non-human organism of the invention
(i) for heterologous reconstitution of a terpene biosynthetic pathway;
(ii) for producing an industrial product, preferably a flavour or fragrance, a biofuel, a fuel composition, a fuel compound, e.g., a blowing agent for diesel fuel compositions, a pesticide, an insect repellent, or an antimicrobial;
(iii) for producing an aliphatic and/or aromatic monoterpene ester from a monoterpene alcohol, preferably from a tertiary monoterpene alcohol;
(iv) for detoxifying a monoterpene alcohol in a microorganism such as bacteria or fungi (e.g. yeast), thereby increasing monoterpene production in said microorganism;
(v) in combination with a GPP synthase and/or S- or R- linalool synthase;
(vi) for increasing the beneficial effects of acetylation in that the hydrophobic acetate partitions more readily go into an organic phase, as compared to the monoterpene alcohol;
(vii) for expressing the alcohol acyl transferase of the invention such that the ratio of monoterpene acetate to monoterpene alcohol is greater than 5:1 or 10:1;
(viii) in a microbial production system for monoterpene esters.

The use of the invention for increasing the beneficial effects of acetylation in that the hydrophobic acetate partitions more readily goes into an organic phase, as compared to the monoterpene alcohol (see item vi) above), can be tested, e.g., by comparison of the IogP value of the monoterpene alcohol and the monoterpene ester.

For instance, the IogP value for linalool is 2.970 (see http://www.thegoodscentscompany.com/data/rw1007872.html#tophyp), and for linalyl acetate 3.930 (see http://www.thegoodscentscompany.com/data/rw1007892.html#tophyp).

For instance, in order to produce the monoterpene ester described herein using fermentation, a two-phase system can be utilized. The monoterpene ester product is extracted into the oil layer whilst the microbe remains in the aqueous layer. The more hydrophobic the molecule is, the higher percentage (%) partitions into this second layer. This percentage can be critical if it makes the difference between crossing a toxic threshold in aqueous layer or not. A two-phase fermentation process for the production of an organic compound is described, e.g., in WO 2015/002528.

The invention also relates to a kit comprising the alcohol acyl transferase of the invention, the nucleic acid of the invention, the vector and/or gene construct of the invention, the host cell of the invention, and/or the transgenic non-human organism of the invention, and optionally at least one monoterpene alcohol, preferably a tertiary monoterpene alcohol. Preferably, said kit is for carrying out the methods of the invention for preparing a monoterpene ester, such as linalyl acetate.

The term "kit" as used herein refers to a collection of means comprising the alcohol acyl transferase of the invention, the nucleic acid of the invention, the vector or gene construct of the invention, the host cell of the invention, and/or the transgenic non-human organism of the invention which are provided in separate or common vials in a ready to use manner for carrying out the methods of the invention as defined herein. The kit can optionally comprise at least one monoterpene alcohol, preferably a tertiary monoterpene alcohol, for instance, as a substrate for the alcohol acyl transferase of the invention. In an aspect, the kit comprises additional means for carrying out said methdos, such as further enzymes, e.g., a S- or L-linalool synthase and/or a GPP synthase, as defined herein. Furthermore, in an aspect, the kit comprises instructions for carrying out the methods of the invention. These instructions can be provided as a manual. In addition, the invention pertains to a monoterpene ester produced by the alcohol acyl transferase of the invention, or the method for preparing a monoterpene ester of the invention, preferably produced from a tertiary monoterpene alcohol, and to a composition comprising said monoterpene ester. Optionally, the composition comprises the alcohol acyl transferase of the invention and, further optionally, at least one monoterpene alcohol, preferably a tertiary monoterpene alcohol. Preferably, the composition is cell-free and/or a composition not found in nature.

The invention also relates to linalyl acetate produced by the alcohol acyl transferase of the invention, or the method for preparing linalyl acetate, of the invention, preferably produced from linalool, and to a composition comprising said linalyl acetate. Optionally, the composition comprises the alcohol acyl transferase of the invention and, further optionally, linalool. Preferably, the composition is cell-free and/or a composition not found in nature.

Further, the invention relates to a composition comprising the alcohol acyl transferase of the invention, and at least one monoterpene alcohol, preferably a tertiary monoterpene alcohol. The alcohol acyl transferase may be in an active state or in a state that its activity can be activated, e.g., by pH change or removal of an inhibitor and the like.

The invention also relates to a fermentation broth, preferably a fermentation broth in a biphasic fermentation system, wherein the fermentation broth comprises the alcohol acyl transferase of the invention, the nucleic acid of the invention, the vector or gene construct of the invention, the host cell of the invention, and/or the non-human transgenic organism of the invention, and one or more monoterpene alcohols and/or one or more monoterpene esters produced by the alcohol acyl transferase of the invention or the methods of the invention.

The monoterpene ester(s) produced by any one of the methods of the invention can be used per se. For instance, linalyl acetate can be used as an additive, or as blowing agent in diesel fuel compositions, as set forth elsewhere herein.

In other embodiments, the monoterpene ester(s) produced by any one of the methods of the invention can be used as starting material for another compound, e.g., a fuel and/or biolubricant compound or composition. For instance, said monoterpene ester(s) can be converted to a fuel and / or biolubricant compound.

Accordingly, the invention further pertains to a method for the production of a fuel and/or biolubricant compound, wherein the method comprises the steps of:
a) Producing one or more monoterpene esters by any one of the methods of the invention;
b) optionally, purifying the one or more monoterpene esters produced in step a); and
c) converting the one or more monoterpene esters of step a), or the optionally purified one or more monoterpene esters of step b), to a fuel and / or biolubricant compound.

Preferably, the fuel and / or biolubricant compounds is selected from the group consisting of:
i. tetrahydrolinalool;
ii. 2,6-dimethyloctane (DMO);
iii. saturated C20 hydrocarbon dimers;
iv. saturated C30 hydrocarbon trimers; and
v. hydrogenated methylcyclopentadiene dimers.

The invention further provides a method for the production of a fuel and / or biolubricant composition, wherein the method comprises the steps of:
a) Producing one or more monoterpene esters by any one of the methods of the invention;
b) optionally, purifying the one or more monoterpene esters produced in step a); and
c) converting part or all of the one or more monoterpene esters of step a), or converting part or all of the optionally purified one or more monoterpene esters of step b), to one or more fuel and / or biolubricant compounds; and
d) combining the one or more fuel and / or biolubricant compounds with additional compounds suitable for a fuel and / or biolubricant composition and, optionally, with one or more monoterpene esters produced in step a) to form a fuel and / or biolubricant composition.

Such additional compounds suitable for a fuel and / or biolubricant composition are known in the art; see, e.g., US 9,816,043 or US 9,802,873.

Preferably, the at least one or more fuel and / or biolubricant compounds is selected from the group consisting of:
i. tetrahydrolinalool;
ii. 2,6-dimethyloctane (DMO);
iii. saturated C20 hydrocarbon dimers;
iv. saturated C30 hydrocarbon trimers; and
v. hydrogenated methylcyclopentadiene dimers.

Preferably, the fuel and / or biolubricant composition comprises in sum between and including 0.01 % (w/w) to 99.99 % (w/w), preferably between and including 0.1 % (w/w) and 99.9 % (w/w), of one or more fuel and / or biolubricant compounds produced from the one or more monoterpene esters and of one or more monoterpene esters obtainable by any one of the methods of the invention.

The invention also pertains to a method for the production of a fuel composition, wherein the method comprises the steps of:
a) Producing linalyl acetate by any one of the methods of the invention;
b) optionally, purifying the linalyl acetate produced in step a); and
c) producing a fuel composition by combining the linalyl acetate of step a), or the optionally purified linalyl acetate of step b), with additional compounds suitable for a fuel composition;
wherein the fuel composition comprises between and including 0.01 % (w/w) to 99.99 % (w/w), preferably between and including 0.1 % (w/w) and 99.9 % (w/w), of linalyl acetate.

Preferably, the fuel composition is a diesel fuel composition and linalyl acetate is used as a blowing agent, as described elsewhere herein. Typically, about 0.5 % (w/w) of linalyl acetate are included in the fuel compositon prepared by this method of the invention.

The method for preparing a monoterpene ester, comprising esterifying a monoterpene alcohol to a monoterpene ester, in the presence of an alcohol acyl transferase of the invention has been described elsewhere herein. This applies mutatis mutandis to the method for preparing linalyl acetate, comprising esterifying linalool to linalyl acetate, in the presence of an alcohol acyl transferase of the invention. The definitions and embodiments apply mutatis mutandis to the method for the production of a fuel and / or biolubricant compound or composition, of the invention.

Preferably, the one or more monoterpene esters are produced from a tertiary monoterpene alcohol, more preferably from linalool, perillyl aclohol and/or alpha terpineol, and most preferably from linalool and / or alpha terpineol.

It is preferred that step a) of these methods of the invention is performed in a fermentation system, preferably a biphasic system, that uses bio-based substrates for the provision of one or more monoterpene alcohols. A bio-based substrate is a substrate intentionally made from substances derived from living (or once-living) organisms.
Preferably, optional step b) of these methods of the invention is used as necessary for further purification of the fermentatively produced monoterpene ester(s) before conversion to one or more fuel or biolubricant compounds, preferably selected from the group consisiting of:
i. tetrahydrolinalool;
ii. 2,6-dimethyloctane (DMO);
iii. saturated C20 Hydrocarbon dimers;
iv. saturated C30 Hydrocarbon trimers;
v. hydrogenated methylcyclopentadiene dimers;
vi. saturated high density multi-cyclic hydrocarbon compounds suitable for missile propulsion; and
vii. hydrogenated C40+ oligomers suitable to produce biolubricant additives.

If bio-based substrates are used in the fermentation, and the monoterpene esters produced by the fermentation are then used for production of said fuel and / or biolubricant compound(s), the resulting fuel and / or lubricant composition comprising said fuel and / or biolubricant compound(s) is at least in part a bio-based fuel and/or biolubricant composition, which is advantegous with respect to the carbon footprint of transport, traffic and aviation.

As example for a hydrogenated methylcyclopentadiene dimer, RJ-4, is shown here: 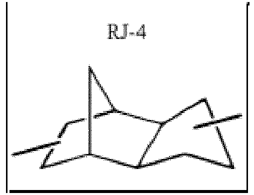 US 9,816,043 describes methods for manufacturing fuels and biolubricant additives. US 9,802,873 describes methods for the conversion of linalool into a drop-in, high-density fuel suitable for ramjet or missile propulsion. Biolubricants are described, e.g., in https://www.sciencedirect.com/topics/agricultural-and-biological-sciences/biolubricants.

The fuel and / or biolubricant composition produced by the method of the invention can be used as gasoline, kerosene fuel, jet fuel, missile fuel, heavy diesel fuel, ship fuel and / or as lubricant. Advantages of fuel compositions produced by the method of the invention are improved carbon dioxide footprint, reduced sulfur content, reduced particulate emissions from the engine, and lower NOx emissions in both diesel and turbine applications.

The invention pertains to fuel compositions that are gasoline, kerosene fuel, jet fuel, missile fuel, heavy diesel fuel or ship fuel largely based on the monoterpene esters produced by the methods of the invention.

Preferably, the fuel composition is a biofuel composition; see, e.g., https://doi.org/10.1016/B978-0-12-815162-4.00011-2.

In another aspect of the invention, the linalyl acetate produced by the inventive methods can be used as blowing agent in diesel fuel compositions, as disclosed, e.g., in WO 2019/201630. The use of the fermentative methods described herein for generating linalyl acetate have the advantage that the fermentative production supplies a bio-based linalyl acetate of defined quality and with low impact on natural resources, for example without the need to isolate it from high value plants like lavender or employ chemical synthesis with a detrimental carbon footprint.

Also, the invention pertains to an industrial product, preferably a flavour or fragrance, a biofuel, a fuel composition, a fuel compound, e.g., a blowing agent for diesel fuel compositions, a pesticide, an insect repellent or an antimicrobial, comprising the monoterpene ester produced by the alcohol acyl transferase of the invention or the methods of the invention, preferably from a tertiary monoterpene alcohol. Such industrial product can at least in part be considered to be based on renewable resources when the monoterpene ester fermentatively produced by the alcohol acyl transferase of the invention or the inventive methods are employed. Moreover, substances of natural or nature-like production are considered more attractive than petroleum-based, synthetic alternatives in many markets.

The invention further pertains to a microbial production system for one or more monoterpene esters, wherein the microbial production system comprises:
a. cells of one or more microbe(s);
b. one or more alcohol acyl transferase(s) of the invention;
c. at least one monoterpene alcohol, preferably a tertiary monoterpene alcohol;
d. acetyl-CoA in an amount suitable for the alcohol acyl transferase(s) of the invention to be active;
e. an aqueous medium suitable for the one or more alcohol acyl transferase(s) of the invention to be active; and
f. optionally, a non-aequeous solvent in which at least one monoterpene ester produced by the alcohol acyl transferase(s) of the invention is miscible.

The at least one monoterpene alcohol, preferably a tertiary monoterpene alcohol, and/or the acetyl-CoA may be added and/or produced by the cells of the one or more microbe(s). Preferably, the microbes are bacteria or fungi as set forth elsewhere herein, more preferably the bacteria is Rhodobacter.

Preferably, the microbial production system of the invention is performed in a fermentation system, preferably a biphasic system, as explained elsewhere herein.

Advantageously, the microbial production system according to the invention can be used industrially in the fermentative production of monoterpene esters as defined herein.

The definitions and embodiments as regards the fermentative production of monoterpene esters in a host cell or non-human transgenic organism of the invention apply mutatis mutandis to the microbial production system of the invention.

In one embodiment, the microbial production system can comprise microbial cells that are inducible for the production of at least one monoterpene alcohol, preferably a tertiary monoterpene alcohol, and/or the acetyl-CoA and/or the one ore more alcohol acyl transferase(s) of the invention.

Preferably, the microbial production system includes means to inactivate or destroy the microbes when sufficient amounts of the at least one monoterpene alcohol, preferably a tertiary monoterpene alcohol, and/or the acetyl-CoA and/or the one ore more alcohol acyl transferase(s) of the invention, and/or one or more monoterpene ester(s) has been produced.

Microbial production system for monoterpenes are known in the art; see, e.g., Zhang et al., Biotechnol Adv. 2017 Dec;35(8):1022-1031. doi: 10.1016/j.biotechadv.2017.09.002. Epub 2017 Sep 6; Cao et al., Appl Microbiol Biotechnol. 2018 Feb;102(4):1535-1544. doi: 10.1007/s00253-017-8695-5. Epub 2017 Dec 20.

Suitable fermentation conditions can be based on known methodology for Rhodobacter, e.g. described in WO 2011/074954 or in WO 2014/014339.

### Sequences

SEQ ID NO: 1 corresponds to the nucleotide sequence of the alcohol acyl transferase (AAT) from Citrus bergamia (AAT9-1-c).
SEQ ID NO: 2 corresponds to the amino acid sequence of the alcohol acyl transferase (AAT) from Citrus bergamia (AAT9-1-c).
SEQ ID NO: 3 corresponds to the amino acid sequence of the alcohol acyl transferase (AAT) from Lavandula angustifolia (10056).
SEQ ID NO: 4 corresponds to the amino acid sequence of the alcohol acyl transferase (AAT) from Lavandula angustifolia (1461).
SEQ ID NO: 5 corresponds to corresponds to the nucleotide sequence of the alcohol acyl transferase (AAT) from Citrus bergamia (AAT9-1-a).
SEQ ID NO: 6 corresponds to the amino acid sequence of the alcohol acyl transferase (AAT) from Citrus bergamia (AAT9-1-a).
SEQ ID NO: 7 corresponds to the nucleotide sequence of the alcohol acyl transferase (AAT) from Citrus bergamia (AAT9-2-a).
SEQ ID NO: 8 corresponds to the amino acid sequence of the alcohol acyl transferase (AAT) from Citrus bergamia (AAT9-2-a).
SEQ ID NO: 9 corresponds to the nucleotide sequence of primer AAT9-1fw.
SEQ ID NO: 10 corresponds to the nucleotide sequence of primer AAT9-2fw.
SEQ ID NO: 11 corresponds to the nucleotide sequence of primer AAT9-1re.
SEQ ID NO: 12 corresponds to the nucleotide sequence of primer AAT9-2re.

### Figures

**Figure 1** shows multiple sequence alignments of the amino acid sequences of variant AAT9-1-c (SEQ ID NO: 2), variant AAT9-1-a (SEQ ID NO: 6), and variant AAT9-2-a (SEQ ID NO: 8), of the alcohol acyl transferase (AAT) from Citrus bergamia.
**Figure 2** shows the nucleotide sequence of variant AAT9-1-c of the alcohol acyl transferase (AAT) from Citrus bergamia (SEQ ID NO: 1), and the amino acid sequence of variant AAT9-1-c (SEQ ID NO: 2).
**Figure 3** GC FID chromatograms show conversion of linalool to linalyl acetate in alcohol acyl transferase (AAT) variant AAT9-1-c (SEQ ID NO: 2) from Citrus bergamia, and not in empty vector pACYCDUET-1 (control).
**Figure 4** shows an alignment (using the NEEDLE algorithm with standard settings) of the amino acid sequence of the alcohol acyl transferase (AAT) from Lavandula angustifolia (termed 10056) (SEQ ID NO: 3), and the other amino acid sequence of the alcohol acyl transferase (AAT) from Lavandula angustifolia (termed 1461) (SEQ ID NO: 4).
**Figure 5** shows multiple sequence alignments of the amino acid sequences of variant AAT9-1-c (SEQ ID NO: 2) from Citrus bergamia, the alcohol acyl transferase (AAT) from Lavandula angustifolia (10056) (SEQ ID NO: 3), and the amino acid sequence of the alcohol acyl transferase (AAT) from Lavandula angustifolia (1461) (SEQ ID NO: 4). The MEIE motif can be seen at the start of SEQ ID NO: 3 and 4.

The invention will now be illustrated by the following examples which shall, however, not be construed as limiting the scope of the present invention.

### Examples

### Example 1: Cloning of the alcohol acyl tranferase from Citrus bergamia

A *Citrus bergamia* fruit of the pre-ripening stage of about 5 cm in diameter was obtained in an orchard in Calabria, Italy. The outside of the peel of the fruit (flavedo) was collected using a zester. 0.5 g of plant material was weighed in a pre-cooled glass tube, and 2 mL of dichloromethane was added. The suspension was vortexed for 1 min, sonicated for 5 min in an ultrasonic bath and centrifuged for 5 min at 1500 g at room temperature. The supernatant was collected and filtered over a column of 1 g sodium sulphate. About 2 µL was analysed by GC/MS using a gas chromatograph, as described in detail by Cankar et al. (Biotechnol J. 2015 Jan; 10(1):180-9. doi: 10.1002/biot.201400288. Epub 2014 Sep 18.). Linalyl acetate was identified by the comparison of retention times and mass spectra to those of an original standard of racemic linalyl acetate (Sigma-Aldrich). This tissue was further taken for extraction of RNA.

The RNA of *Citrus bergamia* root material was isolated as follows: About 15 mL extraction buffer (2% hexadecyl-trimethylammonium bromide, 2% polyvinylpyrrolidinone K 30, 100 mM Tris-HCI (pH 8.0), 25 mM EDTA, 2.0 M NaCl, 0.5 g/L spermidine and 2% β-mercaptoethanol) was warmed to 65 °C, after which 3 g ground tissue was added and mixed. The mixture was extracted two times with an equal volume of chloroform:isoamylalcohol (1 : 24), and one-fourth volume of 10 M LiCI was added to the supernatant and mixed. The RNA was precipitated overnight at 4 °C and harvested by centrifugation at 10 000 g for 20 min. The pellet was dissolved in 500 µL of SSTE [1.0 M NaCl, 0.5% SDS, 10 mM Tris-HC1 (pH 8.0), 1 mM EDTA (pH 8.0)] and extracted once with an equal volume of chloroform: isoamylalcohol. Two volumes of ethanol were added to the supernatant, incubated for at least 2 h at -20 °C, centrifuged at 13 000 g and the supernatant removed. The pellet was air-dried and resuspended in water. Total RNA (60 µg) was shipped to Vertis Biotechnology AG (Freising, Germany). PolyA+ RNA was isolated, random primed cDNA synthesized using a randomized N6 adapter primer and M-MLV H-reverse transcriptase. cDNA was sheared and fractionated, and fragments of a size of 500 bp were used for further analysis. The cDNAs carry attached to their 5'- and 3'-ends the adaptor sequences A and B, as specified by Illumina. The material was subsequently analysed on an Illumina HiSeq Sequencing device. In total, 93,001,205 sequences were read by the HiSeq. Trimmomatic-0.32 was used to trim sequences from Illumina sequencing adapters, Seqprep was used to overlap paired end sequences, and bowtie2 (version 2.2.1) was used to remove phiX contamination (phiX DNA is used as a spike-in control, usually present in <1%). Paired end reads and single reads were used in a Trinity assembly (trinityrnaseq-2.0.2). A total number of 191,426 contigs were assembled by Trinity.

In order to identify alcohol acyltransferases, the *Citrus bergamia* contigs were used to create a database of cDNA sequences. In this database, the TBLASTN program was deployed to identify cDNA sequences that encode proteins that show identity with protein sequences of alcohol acyl transferases (AAT), in particular from Rosa hybrida (AAW31948.1). In total 14 contigs in the C. bergamea cDNA database were identified which have significant homology to alcohol acyl transferases (AAT), and encode a full-length protein. These 14 contigs were further characterised by analyzing them using the BLASTX program to align them to protein sequences present in the UniProt database (downloaded Aug 28, 2019).

Full length open reading frames were amplified from the cDNA of *Citrus bergamia.* Forward and reverse primers as shown in Table 1 were designed and used to amplify total open reading frames in such a way that the reading frame was fused to the C-terminus of a His-6 tag in the plasmid pACYC-DUET-1 (Novagen). The cloned variants were analysed by sequencing the alcohol acyl transferase (AAT) insert. A total of 37 different alcohol acyl transferase (AAT) open reading frames (ORFs) were cloned. Using the primers AAT9-1 fw (SEQ ID NO: 9) and AAT9-1 re (SEQ ID NO: 11), and primers AAT9-2 fw (SEQ ID NO: 10) and AAT9-2 re (SEQ ID NO: 12) (see Table 1), three different closely related cDNAs were obtained.

**Table 1: Primers for cloning of alcohol acyl transferase (AAT) sequences**

| Name | Sequence | Clones |
|---|---|---|
| AAT9-1 fw | taagtataagaaggagatatacatATGAAAATCGATGTTGAAACAATC (SEQ ID NO: 9) | AAT9-1a, AAT9-1c |
| AAT9-2 fw | taagtataagaaggagatatacatATGAAAATCAGTGTCGAAACAATC (SEQ ID NO: 10) | AAT9-2a |
| AAT9-1 re | tttaccagactcgagggtaccCTAAGTGGAAACATAAGCAAG (SEQ ID NO: 11) | AAT9-1a, AAT9-1c |
| AAT9-2 re | tttaccagactcgagggtaccCTAAGGGGAAACATAAGCAAG (SEQ ID NO: 12) | AAT9-2a |

The three different alcohol acyl transferase (AAT) variants obtained with primer pair AAT9-1fw (SEQ ID NO: 9) and AAT9-1re (SEQ ID NO: 11) from Citrus bergamia show the following sequences:
SEQ ID NO: 1 corresponds to the nucleotide sequence of the alcohol acyl transferase (AAT) from Citrus bergamia (variant AAT9-1-c). SEQ ID NO: 2 corresponds to the amino acid sequence of the alcohol acyl transferase (AAT) from Citrus bergamia (variant AAT9-1-c AAT9-1-c).
SEQ ID NO: 5 corresponds to corresponds to the nucleotide sequence of the alcohol acyl transferase (AAT) from Citrus bergamia (variant AAT9-1-a). SEQ ID NO: 6 corresponds to the amino acid sequence of the alcohol acyl transferase (AAT) from Citrus bergamia (variant AAT9-1-a).
SEQ ID NO: 7 corresponds to the nucleotide sequence of the alcohol acyl transferase (AAT) from Citrus bergamia (variant AAT9-2-a). SEQ ID NO: 8 corresponds to the amino acid sequence of the alcohol acyl transferase (AAT) from Citrus bergamia (variant AAT9-2-a).

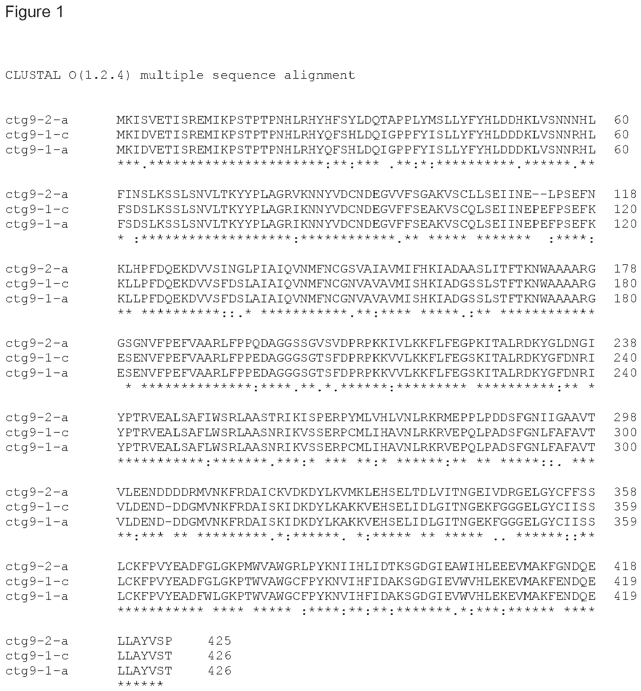

Figure 1 shows multiple sequence alignments of the amino acid sequences of variant AAT9-1-c (SEQ ID NO: 2), variant AAT9-1-a (SEQ ID NO: 6), and variant AAT9-2-a (SEQ ID NO: 8), of the alcohol acyl transferase (AAT) from Citrus bergamia.

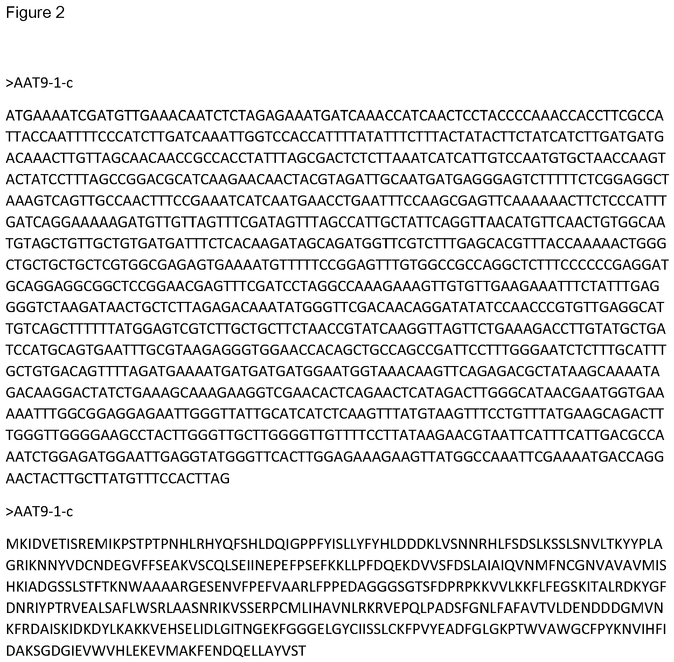

Figure 2 shows the nucleotide sequence of variant AAT9-1-c of the alcohol acyl transferase (AAT) from Citrus bergamia (SEQ ID NO: 1), and the amino acid sequence of variant AAT9-1-c (SEQ ID NO: 2).

### Example 2: Activity of the alcohol acyltransferase variant AAT9-1-c (SEQ ID NO: 2) from Citrus bergamia

The three different alcohol acyl transferase (AAT) variants obtained with primer pair AAT9-1fw (SEQ ID NO: 9) and AAT9-1re (SEQ ID NO: 11) from Citrus bergamia having SEQ ID NO: 2, 6, and 8, were subsequently tested for their ability of converting linalool to linalyl acetate. To this end, the three different variants, and an empty pACYC-DUET-1, were introduced into chemical competent E. coli BL21-RIL (Stratagene), by heat shock transformation, and selected on LB-agar with 1% glucose and 50 ul/ml chloramphenicol. Transformants were transferred to 5 ml LB liquid medium with 1% glucose and 50 ug/ml chloramphenicol and grown overnight at 37°C and 250 rpm.

200 µL of those cultures was transferred to 20 mL of LB medium with the appropriate antibiotic in a 100 mL Erlenmeyer flask, and incubated at 37°C, 250 rpm until the A600 was 0.4 to 0.6. Subsequently, 1 mM IPTG and 2 ml dodecane supplemented with 5 mM linalool (racemic) were added, and cultures were incubated overnight at 18°C and 250 rpm. The next day, dodecane layer was recovered by centrifugation (10 min at 8000xg), diluted in ethyl acetate and analysed by GC FID.

Interestingly, alcohol acyl transferase (AAT) variant AAT9-1-c (SEQ ID NO: 2) displayed a marked reduction of linalool (Rt=11.15 min), and occurrence of a peak corresponding to linalyl acetate (15.45 min) (Figure 3). Apparently, linalool was converted to linalyl acetate when AAT9-1-c was expressed. In one experiment, about 65% by mass of linalool was esterified, in another experiment, about 78% by mass of linalool was esterified, according to preliminary estimates by the present inventors.

Such conversion could not be observed when empty vector was expressed, or when AAT9-1-a (SEQ ID NO: 6) or AAT9-2-a (SEQ ID NO: 8) were expressed.

Having established that AAT9-1-c (SEQ ID NO: 2) is active on linalool, a set of monoterpene alcohols and sesquiterpene alcohols was tested in the presence of E. coli cells expressing AAT9-1-c or empty pACYCDUET-1. Activity of AAT9-1-c (SEQ ID NO: 2) was observed on monoterpene alcohols geraniol, alpha terpineol and verbenol, and corresponding esters could be observed.

Sesquiterpene alcohols nerolidol, patchoulol, (-)-alpha-bisabolol and cedrol were not observed to be converted to their corresponding esters.

Thus, AAT9-1-c (SEQ ID NO: 2) appears to be a monoterpene alcohol-specific acyltransferase, which accepts primary, secondary and tertiary alcohols. This result is particularly surprising because, for instance, linalool is not among the accepted substrates of the AAT enzymes described in the art. The exceptional property of linalool, defining it as a non-substrate for these AATs, could be caused by the position of the alcohol group in linalool, which can be regarded as a tertiary alcohol: The carbon to which the acceptor alcohol group is attached is bonded to three carbon groups. Tertiary alcohols are often difficult to access by enzyme active site pockets, possibly because of the steric accessibility of the alcohol group. Advantageously, the alcohol acyltransferase variant AAT9-1-c (SEQ ID NO: 2) from Citrus bergamia is able to esterify linalool to linalyl acetate, as demonstrated in Figure 3.

### Example 3: Sequence comparisons to alcohol acyltransferase variant AAT9-1-c (SEQ ID NO: 2) from Citrus bergamia

A protein sequence alignment was made between alcohol acyltransferase variant AAT9-1-c (SEQ ID NO: 2) from Citrus bergamia, and its inactive variants AAT9-1-a (SEQ ID NO: 6) and AAT9-2-a (SEQ ID NO: 8) (Figure 1). AAT9-1-c (SEQ ID NO: 2) and AT9-1-a (SEQ ID NO: 6) differ in only one amino acid at position 371, and thus share an identity of 99.77%. AAT9-1c (SEQ ID NO: 2) is 81.60% identical to AAT9-2-a (SEQ ID NO: 8).

A BLASTP analysis of the AAT9-1-c sequence (SEQ ID NO: 2) against the SWISSPROT database (update 2020/09/15) of characterized proteins reveal that the closest characterized proteins encode the Stemmadenine O-acetyltransferase (CrSAT) from Catharanthus roseus (A0A2P1GlW7.1; 34.3% identical), the Vinorine synthase from Rauvolfia serpentine (Q70PR7.2; 35.7%), the Minovincinine 19-hydroxy-O-acetyltransferase from Catharanthus roseus (Q8GZU0.1; 35.4%) and the Salutaridinol 7-O-acetyltransferase (salAT) from Papaver somniferum (Q94FT4.1; 34.5%). Each of these enzymes is involved in acetylation of highly complex alkaloid molecules (compared to linalool), and the inventors define them as part of the vinorine synthase family. This finding could not be expected because the novel alcohol acyltransferase enzyme of the invention is most closely related to a family of alcohol acyl transferase (AATs) which acts on very complex phenolic substances, such as salutaridinol.

A BLASTP analysis of the AAT9-1-c sequence (SEQ ID NO: 2) against the GenBank database (update 2020/09/14) reveal that the most closely related sequences encode proteins from Citrus species, such as those annotated as the vinorine synthase-like from Citrus sinensis (XP_006493396.1; 88.5%), the hypothetical protein CUMW_168950 from Citrus unshiu (GAY56063.1; 88.3%), hypothetical protein CISIN_1g044243mg from Citrus sinensis (KDO41287.1; 82.16%) and the vinorine synthase from Citrus clementina (XP_006423966.1; 81.9%). Clearly, a function for these proteins other than by their homology to alcohol acyltransferases has not yet been identified.

### Example 4: Identification of linalool acetyltransferases in Lavandula angustifolia

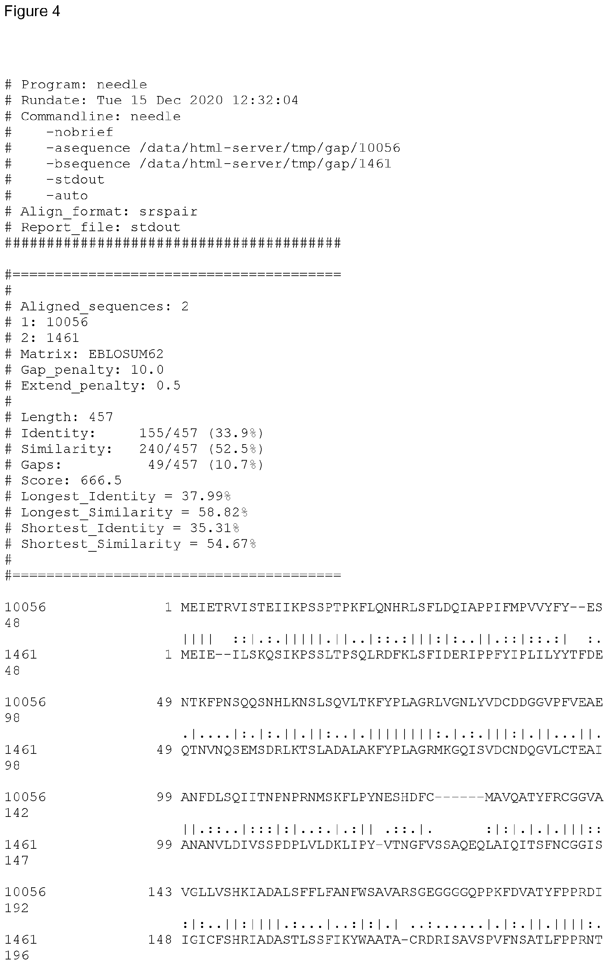

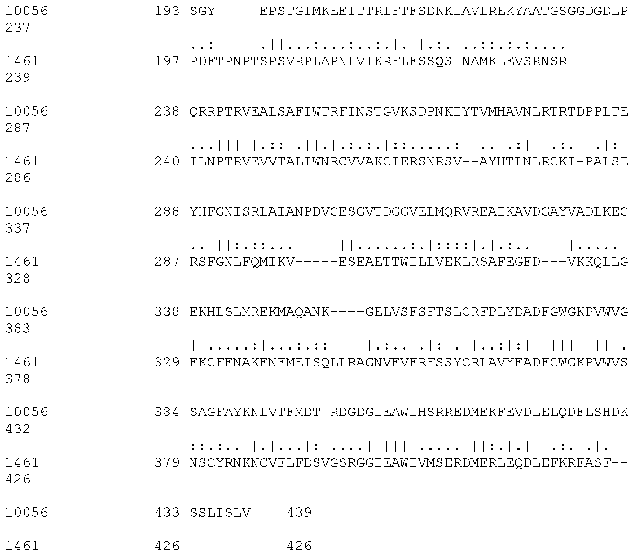

The inventors reasoned that also in Lavandula, like in bergamot, members of the vinorine synthase family were candidates for linalool acetyltransferases. Therefore, sequence data present in the Transcriptome Shotgun Assembly of BioProject PRJNA391145 (https://www.ncbi.nlm.nih.gov/bioproject/?term=txid1196215[Organism:noexp]) were queried by TBLASTN using the RhAAT protein sequence (AAW31948.1), and a set of 16 proteins was revealed. Among these, two protein sequences had significant homology to AAT9-1-c (SEQ ID NO: 2), encoded by ctg1461 (41.9% identical) and ctg10056 (35.3%). Manual editing of the predicted, incomplete protein sequences resulted in sequences that were considered as candidate linalool alcohol acyltransferases. SEQ ID NO: 3 shows the amino acid sequence of the alcohol acyl transferase (AAT) from Lavandula angustifolia (10056). SEQ ID NO: 4 shows the amino acid sequence of the alcohol acyl transferase (AAT) from Lavandula angustifolia (1461). The mentioned sequences are depicted in Figure 4.

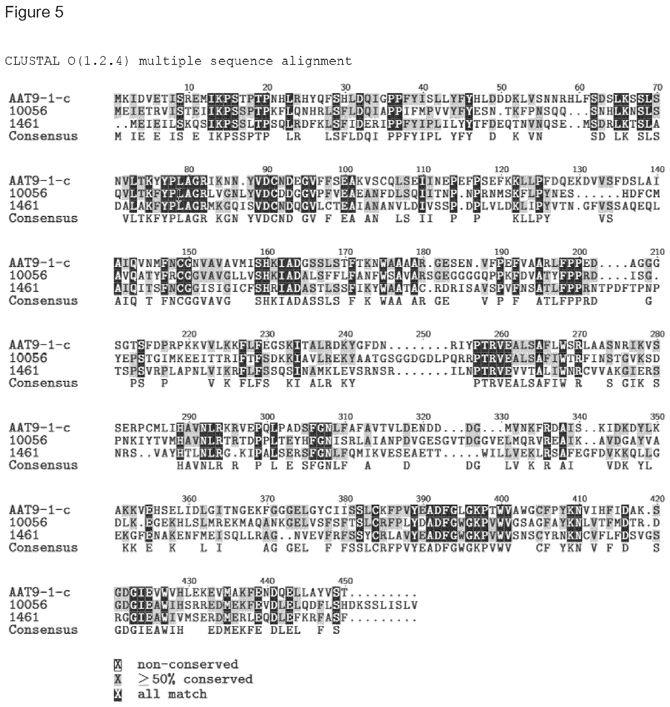

Figure 5 shows multiple sequence alignments of the amino acid sequences of variant AAT9-1-c (SEQ ID NO: 2) from Citrus bergamia, the alcohol acyl transferase (AAT) from Lavandula angustifolia (10056) (SEQ ID NO: 3), and the amino acid sequence of the alcohol acyl transferase (AAT) from Lavandula angustifolia (1461) (SEQ ID NO: 4).

## Claims

1. An alcohol acyl transferase which is capable of esterifying a tertiary monoterpene alcohol such that at least 30% by mass of said tertiary monoterpene alcohol is esterified, preferably within 36 h, 24 h, 12 h, 6 h, 3 h, 2 h, 1h, 45 min or 30 min, more preferably in a microbial cell.

2. The alcohol acyl transferase of claim 1, comprising an amino acid sequence selected from the group consisting of:
a) an amino acid sequence as shown in any one of the sequences of SEQ ID NO: 2, SEQ ID NO: 3 (Lavandula AAT-10056) or SEQ ID NO: 4 (Lavandula AAT-1461);
b) an amino acid sequence with at least 40% sequence identity at the amino acid level with SEQ ID NO: 2, or an amino acid sequence with at least 80% sequence identity, or preferably 85% sequence identity, at the amino acid level with SEQ ID NO: 3 (Lavandula AAT-10056) or SEQ ID NO: 4 (Lavandula AAT-1461); and
c) an enzymatically active fragment of the amino acid sequence of a) or b).

3. The alcohol acyl transferase of claim 1 or 2, which is capable of esterifying a tertiary monoterpene alcohol such that at least 50 · g of monoterpene ester per minute and per gram of alcohol acyl transferase is produced, at 30 °C and at a pH in the range of 6.0 to 8.5, and under conditions where the substrates are not limiting.

4. Nucleic acid comprising a nucleic acid sequence encoding the alcohol acyl transferase of claim 1, 2 or 3, or a complementary sequence thereof.

5. A vector or gene construct comprising the nucleic acid of claim 4.

6. A host cell comprising the vector or gene construct of claim 5, wherein the host cell is preferably a bacterial cell, a yeast cell, a fungal cell, an algal cell or a cyanobacterial cell, a non-human animal cell or a non-human mammalian cell, or a plant cell, more preferably a bacterial cell, a yeast cell, or a fungal cell.

7. A transgenic non-human organism comprising the nucleic acid of claim 4, the vector or gene construct of claim 5, or the host cell of claim 6.

8. A method for preparing a monoterpene ester, comprising esterifying a monoterpene alcohol to a monoterpene ester, in the presence of an alcohol acyl transferase of claim 1, 2, or 3.

9. The method of claim 8, wherein the monoterpene alcohol is a primary, secondary or tertiary monoterpene alcohol, preferably a tertiary monoterpene alcohol.

10. A method for preparing linalyl acetate, comprising esterifying linalool to linalyl acetate, in the presence of an alcohol acyl transferase of claim 1, 2, or 3.

11. The method of claim 8, 9, or 10, wherein the alcohol acyl transferase of claim 1, 2, or 3, is used in combination with a GPP synthase and/or a S- or R- linalool synthase.

12. The method of claim 8, 9, or 10, wherein the monoterpene ester of claim 8, or the linalyl acetate of claim 10, is prepared in the host cell of claim 6, or a non-human transgenic organism of claim 7, expressing the alcohol acyl transferase of claim 1, 2, or 3.

13. Use of the alcohol acyl transferase of claim 1, 2, or 3, the nucleic acid of claim 4, the vector or gene construct of claim 5, the host cell of claim 6, or the transgenic non-human organism of claim 7,
(i) for heterologous reconstitution of a terpene biosynthetic pathway;
(ii) for producing an industrial product, preferably a flavour or fragrance, a biofuel, a fuel composition, a fuel compound, a pesticide, an insect repellent or an antimicrobial;
(iii) for producing an aliphatic and/or aromatic monoterpene ester from a monoterpene alcohol, preferably from a tertiary monoterpene alcohol;
(iv) for detoxifying a monoterpene alcohol in a microorganism, thereby increasing monoterpene production in said microorganism, wherein the microorganism is preferably a bacteria or a fungus;
(v) in combination with a GPP synthase and/or a S- or R- linalool synthase;
(vi) for increasing the beneficial effects of acetylation in that the hydrophobic acetate partitions more readily go into an organic phase, in comparison to the monoterpene alcohol;
(vii) for expressing the alcohol acyl transferase of claim 1, 2 or 3 such that the ratio of monoterpene acetate to monoterpene alcohol is greater than 5:1 or 10:1; or
(viii) in a microbial production system for producing monoterpene esters.

14. Kit comprising the alcohol acyl transferase of claim 1, 2 or 3, the nucleic acid of claim 4, the vector and/or gene construct of claim 5, the host cell of claim 6, and/or the transgenic non-human organism of claim 7, and optionally at least one monoterpene alcohol, preferably a tertiary monoterpene alcohol.

15. Method for the production of a fuel and/or biolubricant compound, wherein the method comprises the steps of:
a) Producing one or more monoterpene esters by any one of the methods of claim 8 to 12;
b) optionally, purifying the one or more monoterpene esters produced in step a); and
c) converting the one or more monoterpene esters of step a), or the optionally purified one or more monoterpene esters of step b), to a fuel and / or biolubricant compound.


SEQUENCE LISTING

<110>  Isobionics B.V.

<120>  Enzymes and methods for fermentative production of monoterpene
       esters

<130>  B202673EP

<160>  12

<170>  PatentIn version 3.5

<210>  1
<211>  1281
<212>  DNA
<213>  Citrus bergamia

<400>  1
atgaaaatcg atgttgaaac aatctctaga gaaatgatca aaccatcaac tcctacccca      60

aaccaccttc gccattacca attttcccat cttgatcaaa ttggtccacc attttatatt     120

tctttactat acttctatca tcttgatgat gacaaacttg ttagcaacaa ccgccaccta     180

tttagcgact ctcttaaatc atcattgtcc aatgtgctaa ccaagtacta tcctttagcc     240

ggacgcatca agaacaacta cgtagattgc aatgatgagg gagtcttttt ctcggaggct     300

aaagtcagtt gccaactttc cgaaatcatc aatgaacctg aatttccaag cgagttcaaa     360

aaacttctcc catttgatca ggaaaaagat gttgttagtt tcgatagttt agccattgct     420

attcaggtta acatgttcaa ctgtggcaat gtagctgttg ctgtgatgat ttctcacaag     480

atagcagatg gttcgtcttt gagcacgttt accaaaaact gggctgctgc tgctcgtggc     540

gagagtgaaa atgtttttcc ggagtttgtg gccgccaggc tctttccccc cgaggatgca     600

ggaggcggct ccggaacgag tttcgatcct aggccaaaga aagttgtgtt gaagaaattt     660

ctatttgagg ggtctaagat aactgctctt agagacaaat atgggttcga caacaggata     720

tatccaaccc gtgttgaggc attgtcagct tttttatgga gtcgtcttgc tgcttctaac     780

cgtatcaagg ttagttctga aagaccttgt atgctgatcc atgcagtgaa tttgcgtaag     840

agggtggaac cacagctgcc agccgattcc tttgggaatc tctttgcatt tgctgtgaca     900

gttttagatg aaaatgatga tgatggaatg gtaaacaagt tcagagacgc tataagcaaa     960

atagacaagg actatctgaa agcaaagaag gtcgaacact cagaactcat agacttgggc    1020

ataacgaatg gtgaaaaatt tggcggagga gaattgggtt attgcatcat ctcaagttta    1080

tgtaagtttc ctgtttatga agcagacttt gggttgggga agcctacttg ggttgcttgg    1140

ggttgttttc cttataagaa cgtaattcat ttcattgacg ccaaatctgg agatggaatt    1200

gaggtatggg ttcacttgga gaaagaagtt atggccaaat tcgaaaatga ccaggaacta    1260

cttgcttatg tttccactta g                                               1281

<210>  2
<211>  426
<212>  PRT
<213>  Citrus bergamia

<400>  2

Met Lys Ile Asp Val Glu Thr Ile Ser Arg Glu Met Ile Lys Pro Ser
1               5                   10                  15

Thr Pro Thr Pro Asn His Leu Arg His Tyr Gln Phe Ser His Leu Asp
            20                  25                  30

Gln Ile Gly Pro Pro Phe Tyr Ile Ser Leu Leu Tyr Phe Tyr His Leu
        35                  40                  45

Asp Asp Asp Lys Leu Val Ser Asn Asn Arg His Leu Phe Ser Asp Ser
    50                  55                  60

Leu Lys Ser Ser Leu Ser Asn Val Leu Thr Lys Tyr Tyr Pro Leu Ala
65                  70                  75                  80

Gly Arg Ile Lys Asn Asn Tyr Val Asp Cys Asn Asp Glu Gly Val Phe
                85                  90                  95

Phe Ser Glu Ala Lys Val Ser Cys Gln Leu Ser Glu Ile Ile Asn Glu
            100                 105                 110

Pro Glu Phe Pro Ser Glu Phe Lys Lys Leu Leu Pro Phe Asp Gln Glu
        115                 120                 125

Lys Asp Val Val Ser Phe Asp Ser Leu Ala Ile Ala Ile Gln Val Asn
    130                 135                 140

Met Phe Asn Cys Gly Asn Val Ala Val Ala Val Met Ile Ser His Lys
145                 150                 155                 160

Ile Ala Asp Gly Ser Ser Leu Ser Thr Phe Thr Lys Asn Trp Ala Ala
                165                 170                 175

Ala Ala Arg Gly Glu Ser Glu Asn Val Phe Pro Glu Phe Val Ala Ala
            180                 185                 190

Arg Leu Phe Pro Pro Glu Asp Ala Gly Gly Gly Ser Gly Thr Ser Phe
        195                 200                 205

Asp Pro Arg Pro Lys Lys Val Val Leu Lys Lys Phe Leu Phe Glu Gly
    210                 215                 220

Ser Lys Ile Thr Ala Leu Arg Asp Lys Tyr Gly Phe Asp Asn Arg Ile
225                 230                 235                 240

Tyr Pro Thr Arg Val Glu Ala Leu Ser Ala Phe Leu Trp Ser Arg Leu
                245                 250                 255

Ala Ala Ser Asn Arg Ile Lys Val Ser Ser Glu Arg Pro Cys Met Leu
            260                 265                 270

Ile His Ala Val Asn Leu Arg Lys Arg Val Glu Pro Gln Leu Pro Ala
        275                 280                 285

Asp Ser Phe Gly Asn Leu Phe Ala Phe Ala Val Thr Val Leu Asp Glu
    290                 295                 300

Asn Asp Asp Asp Gly Met Val Asn Lys Phe Arg Asp Ala Ile Ser Lys
305                 310                 315                 320

Ile Asp Lys Asp Tyr Leu Lys Ala Lys Lys Val Glu His Ser Glu Leu
                325                 330                 335

Ile Asp Leu Gly Ile Thr Asn Gly Glu Lys Phe Gly Gly Gly Glu Leu
            340                 345                 350

Gly Tyr Cys Ile Ile Ser Ser Leu Cys Lys Phe Pro Val Tyr Glu Ala
        355                 360                 365

Asp Phe Gly Leu Gly Lys Pro Thr Trp Val Ala Trp Gly Cys Phe Pro
    370                 375                 380

Tyr Lys Asn Val Ile His Phe Ile Asp Ala Lys Ser Gly Asp Gly Ile
385                 390                 395                 400

Glu Val Trp Val His Leu Glu Lys Glu Val Met Ala Lys Phe Glu Asn
                405                 410                 415

Asp Gln Glu Leu Leu Ala Tyr Val Ser Thr
            420                 425

<210>  3
<211>  439
<212>  PRT
<213>  Lavandula angustifolia

<400>  3

Met Glu Ile Glu Thr Arg Val Ile Ser Thr Glu Ile Ile Lys Pro Ser
1               5                   10                  15

Ser Pro Thr Pro Lys Phe Leu Gln Asn His Arg Leu Ser Phe Leu Asp
            20                  25                  30
Gln Ile Ala Pro Pro Ile Phe Met Pro Val Val Tyr Phe Tyr Glu Ser
        35                  40                  45
Asn Thr Lys Phe Pro Asn Ser Gln Gln Ser Asn His Leu Lys Asn Ser
    50                  55                  60
Leu Ser Gln Val Leu Thr Lys Phe Tyr Pro Leu Ala Gly Arg Leu Val
65                  70                  75                  80
Gly Asn Leu Tyr Val Asp Cys Asp Asp Gly Gly Val Pro Phe Val Glu
                85                  90                  95
Ala Glu Ala Asn Phe Asp Leu Ser Gln Ile Ile Thr Asn Pro Asn Pro
            100                 105                 110
Arg Asn Met Ser Lys Phe Leu Pro Tyr Asn Glu Ser His Asp Phe Cys
        115                 120                 125
Met Ala Val Gln Ala Thr Tyr Phe Arg Cys Gly Gly Val Ala Val Gly
    130                 135                 140
Leu Leu Val Ser His Lys Ile Ala Asp Ala Leu Ser Phe Phe Leu Phe
145                 150                 155                 160
Ala Asn Phe Trp Ser Ala Val Ala Arg Ser Gly Glu Gly Gly Gly Gly
                165                 170                 175
Gln Pro Pro Lys Phe Asp Val Ala Thr Tyr Phe Pro Pro Arg Asp Ile
            180                 185                 190
Ser Gly Tyr Glu Pro Ser Thr Gly Ile Met Lys Glu Glu Ile Thr Thr
        195                 200                 205
Arg Ile Phe Thr Phe Ser Asp Lys Lys Ile Ala Val Leu Arg Glu Lys
    210                 215                 220
Tyr Ala Ala Thr Gly Ser Gly Gly Asp Gly Asp Leu Pro Gln Arg Arg
225                 230                 235                 240
Pro Thr Arg Val Glu Ala Leu Ser Ala Phe Ile Trp Thr Arg Phe Ile
                245                 250                 255
Asn Ser Thr Gly Val Lys Ser Asp Pro Asn Lys Ile Tyr Thr Val Met
            260                 265                 270

His Ala Val Asn Leu Arg Thr Arg Thr Asp Pro Pro Leu Thr Glu Tyr
        275                 280                 285

His Phe Gly Asn Ile Ser Arg Leu Ala Ile Ala Asn Pro Asp Val Gly
    290                 295                 300

Glu Ser Gly Val Thr Asp Gly Gly Val Glu Leu Met Gln Arg Val Arg
305                 310                 315                 320

Glu Ala Ile Lys Ala Val Asp Gly Ala Tyr Val Ala Asp Leu Lys Glu
                325                 330                 335

Gly Glu Lys His Leu Ser Leu Met Arg Glu Lys Met Ala Gln Ala Asn
            340                 345                 350

Lys Gly Glu Leu Val Ser Phe Ser Phe Thr Ser Leu Cys Arg Phe Pro
        355                 360                 365

Leu Tyr Asp Ala Asp Phe Gly Trp Gly Lys Pro Val Trp Val Gly Ser
    370                 375                 380

Ala Gly Phe Ala Tyr Lys Asn Leu Val Thr Phe Met Asp Thr Arg Asp
385                 390                 395                 400

Gly Asp Gly Ile Glu Ala Trp Ile His Ser Arg Arg Glu Asp Met Glu
                405                 410                 415

Lys Phe Glu Val Asp Leu Glu Leu Gln Asp Phe Leu Ser His Asp Lys
            420                 425                 430

Ser Ser Leu Ile Ser Leu Val
        435

<210>  4
<211>  426
<212>  PRT
<213>  Lavandula angustifolia

<400>  4

Met Glu Ile Glu Ile Leu Ser Lys Gln Ser Ile Lys Pro Ser Ser Leu
1               5                   10                  15

Thr Pro Ser Gln Leu Arg Asp Phe Lys Leu Ser Phe Ile Asp Glu Arg
            20                  25                  30

Ile Pro Pro Phe Tyr Ile Pro Leu Ile Leu Tyr Tyr Thr Phe Asp Glu
        35                  40                  45

Gln Thr Asn Val Asn Gln Ser Glu Met Ser Asp Arg Leu Lys Thr Ser
    50                  55                  60

Leu Ala Asp Ala Leu Ala Lys Phe Tyr Pro Leu Ala Gly Arg Met Lys
65                  70                  75                  80

Gly Gln Ile Ser Val Asp Cys Asn Asp Gln Gly Val Leu Cys Thr Glu
                85                  90                  95

Ala Ile Ala Asn Ala Asn Val Leu Asp Ile Val Ser Ser Pro Asp Pro
            100                 105                 110

Leu Val Leu Asp Lys Leu Ile Pro Tyr Val Thr Asn Gly Phe Val Ser
        115                 120                 125

Ser Ala Gln Glu Gln Leu Ala Ile Gln Ile Thr Ser Phe Asn Cys Gly
    130                 135                 140

Gly Ile Ser Ile Gly Ile Cys Phe Ser His Arg Ile Ala Asp Ala Ser
145                 150                 155                 160

Thr Leu Ser Ser Phe Ile Lys Tyr Trp Ala Ala Thr Ala Cys Arg Asp
                165                 170                 175

Arg Ile Ser Ala Val Ser Pro Val Phe Asn Ser Ala Thr Leu Phe Pro
            180                 185                 190

Pro Arg Asn Thr Pro Asp Phe Thr Pro Asn Pro Thr Ser Pro Ser Val
        195                 200                 205

Arg Pro Leu Ala Pro Asn Leu Val Ile Lys Arg Phe Leu Phe Ser Ser
    210                 215                 220

Gln Ser Ile Asn Ala Met Lys Leu Glu Val Ser Arg Asn Ser Arg Ile
225                 230                 235                 240

Leu Asn Pro Thr Arg Val Glu Val Val Thr Ala Leu Ile Trp Asn Arg
                245                 250                 255

Cys Val Val Ala Lys Gly Ile Glu Arg Ser Asn Arg Ser Val Ala Tyr
            260                 265                 270

His Thr Leu Asn Leu Arg Gly Lys Ile Pro Ala Leu Ser Glu Arg Ser
        275                 280                 285

Phe Gly Asn Leu Phe Gln Met Ile Lys Val Glu Ser Glu Ala Glu Thr

290                 295                 300

Thr Trp Ile Leu Leu Val Glu Lys Leu Arg Ser Ala Phe Glu Gly Phe
305                 310                 315                 320

Asp Val Lys Lys Gln Leu Leu Gly Glu Lys Gly Phe Glu Asn Ala Lys
                325                 330                 335

Glu Asn Phe Met Glu Ile Ser Gln Leu Leu Arg Ala Gly Asn Val Glu
            340                 345                 350

Val Phe Arg Phe Ser Ser Tyr Cys Arg Leu Ala Val Tyr Glu Ala Asp
        355                 360                 365

Phe Gly Trp Gly Lys Pro Val Trp Val Ser Asn Ser Cys Tyr Arg Asn
    370                 375                 380

Lys Asn Cys Val Phe Leu Phe Asp Ser Val Gly Ser Arg Gly Gly Ile
385                 390                 395                 400

Glu Ala Trp Ile Val Met Ser Glu Arg Asp Met Glu Arg Leu Glu Gln
                405                 410                 415

Asp Leu Glu Phe Lys Arg Phe Ala Ser Phe
            420                 425

<210>  5
<211>  1281
<212>  DNA
<213>  Citrus bergamia

<400>  5
atgaaaatcg atgttgaaac aatctctaga gaaatgatca aaccatcaac tcctacccca      60

aaccaccttc gccattacca attttcccat cttgatcaaa ttggtccacc attttatatt     120

tctttactat acttctatca tcttgatgat gacaaacttg ttagcaacaa ccgccaccta     180

tttagcgact ctcttaaatc atcattgtcc aatgtgctaa ccaagtactg tcctttagcc     240

ggacgcatca agaacaacta cgtagattgc aatgatgagg gagtcttttt ctcggaggct     300

aaagtcagtt gccaactttc cgaaatcatc aatgaacctg aatttccaag cgagttcaaa     360

aaacttctcc catttgatca ggaaaaagat gttgttagtt tcgatagttt agccattgct     420

attcaggtta acatgttcaa ctgtggcaat gtagctgttg ctgtgatgat ttctcacaag     480

atagcagatg gttcgtcttt gagcacgttt accaaaaact gggctgctgc tgctcgtggc     540

gagagtgaaa atgtttttcc ggagtttgtg gccgccaggc tctttccccc cgaggatgca     600

ggaggcggct ccggaacgag tttcgatcct aggccaaaga aagttgtgtt gaagaaattt     660

ctatttgagg ggtctaagat aactgctctt agagacaaat atgggttcga caacaggata 720

tatccaaccc gtgttgaggc attgtcagct tttttatgga gtcgtcttgc tgcttctaac 780

cgtatcaagg ttagttctga aagaccttgt atgctgatcc atgcagtgaa tttgcgtaag 840

agggtggaac cacagctgcc agccgattcc tttgggaatc tctttgcatt tgctgtgaca 900

gttttagatg aaaatgatga tgatggaatg gtaaacaagt tcagagacgc tataagcaaa 960

atagacaagg actatctgaa agcaaagaag gtcgaacact cagaactcat agacttgggc 1020

ataacgaatg gtgaaaaatt tggcggagga gaattgggtt attgcatcat ctcaagttta 1080

tgtaagtttc ctgtttatga agcagacttt tggttgggga agcctacttg ggttgcttgg 1140

ggttgttttc cttataagaa cgtaattcat ttcattgacg ccaaatctgg agatggaatt 1200

gaggtatggg ttcacttgga gaaagaagtt atggccaaat tcgaaaatga ccaggaacta 1260

cttgcttatg tttccactta g 1281

<210> 6
<211> 426
<212> PRT
<213> Citrus bergamia

<400> 6

Met Lys Ile Asp Val Glu Thr Ile Ser Arg Glu Met Ile Lys Pro Ser
1               5                   10                  15

Thr Pro Thr Pro Asn His Leu Arg His Tyr Gln Phe Ser His Leu Asp
            20                  25                  30

Gln Ile Gly Pro Pro Phe Tyr Ile Ser Leu Leu Tyr Phe Tyr His Leu
        35                  40                  45

Asp Asp Asp Lys Leu Val Ser Asn Asn Arg His Leu Phe Ser Asp Ser
    50                  55                  60

Leu Lys Ser Ser Leu Ser Asn Val Leu Thr Lys Tyr Tyr Pro Leu Ala
65                  70                  75                  80

Gly Arg Ile Lys Asn Asn Tyr Val Asp Cys Asn Asp Glu Gly Val Phe
                85                  90                  95

Phe Ser Glu Ala Lys Val Ser Cys Gln Leu Ser Glu Ile Ile Asn Glu
            100                 105                 110

Pro Glu Phe Pro Ser Glu Phe Lys Lys Leu Leu Pro Phe Asp Gln Glu
        115                 120                 125

Lys Asp Val Val Ser Phe Asp Ser Leu Ala Ile Ala Ile Gln Val Asn

130                 135                 140

Met Phe Asn Cys Gly Asn Val Ala Val Ala Val Met Ile Ser His Lys
145                 150                 155                 160

Ile Ala Asp Gly Ser Ser Leu Ser Thr Phe Thr Lys Asn Trp Ala Ala
                165                 170                 175

Ala Ala Arg Gly Glu Ser Glu Asn Val Phe Pro Glu Phe Val Ala Ala
            180                 185                 190

Arg Leu Phe Pro Pro Glu Asp Ala Gly Gly Gly Ser Gly Thr Ser Phe
        195                 200                 205

Asp Pro Arg Pro Lys Lys Val Val Leu Lys Lys Phe Leu Phe Glu Gly
    210                 215                 220

Ser Lys Ile Thr Ala Leu Arg Asp Lys Tyr Gly Phe Asp Asn Arg Ile
225                 230                 235                 240

Tyr Pro Thr Arg Val Glu Ala Leu Ser Ala Phe Leu Trp Ser Arg Leu
                245                 250                 255

Ala Ala Ser Asn Arg Ile Lys Val Ser Ser Glu Arg Pro Cys Met Leu
            260                 265                 270

Ile His Ala Val Asn Leu Arg Lys Arg Val Glu Pro Gln Leu Pro Ala
        275                 280                 285

Asp Ser Phe Gly Asn Leu Phe Ala Phe Ala Val Thr Val Leu Asp Glu
    290                 295                 300

Asn Asp Asp Asp Gly Met Val Asn Lys Phe Arg Asp Ala Ile Ser Lys
305                 310                 315                 320

Ile Asp Lys Asp Tyr Leu Lys Ala Lys Lys Val Glu His Ser Glu Leu
                325                 330                 335

Ile Asp Leu Gly Ile Thr Asn Gly Glu Lys Phe Gly Gly Gly Glu Leu
            340                 345                 350

Gly Tyr Cys Ile Ile Ser Ser Leu Cys Lys Phe Pro Val Tyr Glu Ala
        355                 360                 365

Asp Phe Trp Leu Gly Lys Pro Thr Trp Val Ala Trp Gly Cys Phe Pro
    370                 375                 380

Tyr Lys Asn Val Ile His Phe Ile Asp Ala Lys Ser Gly Asp Gly Ile
385                 390                 395                 400

Glu Val Trp Val His Leu Glu Lys Glu Val Met Ala Lys Phe Glu Asn
                405                 410                 415

Asp Gln Glu Leu Leu Ala Tyr Val Ser Thr
            420                 425

<210>  7
<211>  1278
<212>  DNA
<213>  Citrus bergamia

<400>  7
atgaaaatca gtgtcgaaac aatctctaga gaaatgatca aaccatcaac cccaacccca      60

aatcaccttc gccattacca cttttcctat cttgatcaaa ccgctccgcc attatatatg     120

tctttgctgt acttctatca tcttgatgat cacaaacttg ttagcaacaa caaccaccta     180

ttcattaact ctcttaaatc atcattatcc aatgtgctaa ccaagtacta tcctttagcc     240

ggacgcgtca agaacaacta tgtagattgc aacgatgagg gagtcgtttt ctcggaggct     300

aaagtcagct gtctactttc agaaatcatc aacgaacttc caagtgagtt caacaaactt     360

cacccatttg atcaggaaaa agacgttgtc agtatcaatg gtttacccat tgccattcaa     420

gttaacatgt ttaattgtgg cagtgttgct attgctgtga tgatttttca caagatagca     480

gatgctgcgt ctttgattac gtttaccaaa aactgggctg ctgctgctcg tggcgggagt     540

ggaaatgttt ttccagaatt tgtggccgcc aggctctttc ccccccagga tgcaggtggc     600

agctcaggcg tgagtgtcga tcctaggcca aagaaaattg tgctgaagaa atttctgttt     660

gaggggccta agataactgc tcttagagac aaatatgggt tagacaacgg gatataccca     720

acccgtgttg aggcattgtc agcttttata tggagtcgtc tagcggcttc tactcgtatt     780

aagattagtc ctgaaagacc ttatatgctg gtccatctag tgaatttgcg taagaggatg     840

gaaccaccgc tgccggacga ttcttttggg aatattattg gagctgctgt aacagtttta     900

gaagaaaatg atgatgatga tcgaatggta aacaagttca gagacgctat atgcaaagta     960

gacaaggatt atctgaaagt aatgaagctt gaacactcag aactcaccga cttagtcata    1020

acaaatggtg aaatagttga cagaggagaa ttgggttatt gcttcttctc aagtttatgt    1080

aagtttcctg tatacgaagc agattttggg ttggggaagc ctatgtgggt tgcttggggt    1140

cgtttgcctt ataagaatat aattcatttg attgacacca aatctggaga tggaattgag    1200

gcatggattc acttggagga agaagttatg gccaaatttg gaaatgacca ggagctactt    1260

gcttatgttt ccccttag                                                  1278

<210>  8
<211>  425
<212>  PRT
<213>  Citrus bergamia

<400>  8

Met Lys Ile Ser Val Glu Thr Ile Ser Arg Glu Met Ile Lys Pro Ser
1               5                   10                  15

Thr Pro Thr Pro Asn His Leu Arg His Tyr His Phe Ser Tyr Leu Asp
            20                  25                  30

Gln Thr Ala Pro Pro Leu Tyr Met Ser Leu Leu Tyr Phe Tyr His Leu
        35                  40                  45

Asp Asp His Lys Leu Val Ser Asn Asn Asn His Leu Phe Ile Asn Ser
    50                  55                  60

Leu Lys Ser Ser Leu Ser Asn Val Leu Thr Lys Tyr Tyr Pro Leu Ala
65                  70                  75                  80

Gly Arg Val Lys Asn Asn Tyr Val Asp Cys Asn Asp Glu Gly Val Val
                85                  90                  95

Phe Ser Glu Ala Lys Val Ser Cys Leu Leu Ser Glu Ile Ile Asn Glu
            100                 105                 110

Leu Pro Ser Glu Phe Asn Lys Leu His Pro Phe Asp Gln Glu Lys Asp
        115                 120                 125

Val Val Ser Ile Asn Gly Leu Pro Ile Ala Ile Gln Val Asn Met Phe
    130                 135                 140

Asn Cys Gly Ser Val Ala Ile Ala Val Met Ile Phe His Lys Ile Ala
145                 150                 155                 160

Asp Ala Ala Ser Leu Ile Thr Phe Thr Lys Asn Trp Ala Ala Ala Ala
                165                 170                 175

Arg Gly Gly Ser Gly Asn Val Phe Pro Glu Phe Val Ala Ala Arg Leu
            180                 185                 190

Phe Pro Pro Gln Asp Ala Gly Gly Ser Ser Gly Val Ser Val Asp Pro
        195                 200                 205

Arg Pro Lys Lys Ile Val Leu Lys Lys Phe Leu Phe Glu Gly Pro Lys
    210                 215                 220

Ile Thr Ala Leu Arg Asp Lys Tyr Gly Leu Asp Asn Gly Ile Tyr Pro
225                 230                 235                 240

Thr Arg Val Glu Ala Leu Ser Ala Phe Ile Trp Ser Arg Leu Ala Ala
                245                 250                 255

Ser Thr Arg Ile Lys Ile Ser Pro Glu Arg Pro Tyr Met Leu Val His
            260                 265                 270

Leu Val Asn Leu Arg Lys Arg Met Glu Pro Pro Leu Pro Asp Asp Ser
        275                 280                 285

Phe Gly Asn Ile Ile Gly Ala Ala Val Thr Val Leu Glu Glu Asn Asp
    290                 295                 300

Asp Asp Asp Arg Met Val Asn Lys Phe Arg Asp Ala Ile Cys Lys Val
305                 310                 315                 320

Asp Lys Asp Tyr Leu Lys Val Met Lys Leu Glu His Ser Glu Leu Thr
                325                 330                 335

Asp Leu Val Ile Thr Asn Gly Glu Ile Val Asp Arg Gly Glu Leu Gly
            340                 345                 350

Tyr Cys Phe Phe Ser Ser Leu Cys Lys Phe Pro Val Tyr Glu Ala Asp
        355                 360                 365

Phe Gly Leu Gly Lys Pro Met Trp Val Ala Trp Gly Arg Leu Pro Tyr
    370                 375                 380

Lys Asn Ile Ile His Leu Ile Asp Thr Lys Ser Gly Asp Gly Ile Glu
385                 390                 395                 400

Ala Trp Ile His Leu Glu Glu Glu Val Met Ala Lys Phe Gly Asn Asp
                405                 410                 415

Gln Glu Leu Leu Ala Tyr Val Ser Pro
            420                 425

<210>  9
<211>  48
<212>  DNA
<213>  primer

<400>  9
taagtataag aaggagatat acatatgaaa atcgatgttg aaacaatc                48

<210>  10
<211>  48

<212>  DNA
<213>  primer

<400>  10
taagtataag aaggagatat acatatgaaa atcagtgtcg aaacaatc                    48


<210>  11
<211>  42
<212>  DNA
<213>  primer

<400>  11
tttaccagac tcgagggtac cctaagtgga aacataagca ag                          42


<210>  12
<211>  42
<212>  DNA
<213>  primer

<400>  12
tttaccagac tcgagggtac cctaagggga aacataagca ag                          42